# EUROPEAN PATENT APPLICATION

(11) **EP 4 215 544 A1**
(43) Date of publication of application: **26.07.2023**
(21) Application number: 21866020.7
(22) Date of filing: 09.09.2021
(51) Int. Cl.: C07K 16/10, C12N 15/13, G01N 33/577, G01N 33/569, A61K 39/42, A61P 31/14

(54) **MONOCLONAL ANTIBODY FOR CORONAVIRUS SPIKE PROTEIN, AND USE THEREOF**

(30) Priority: 09.09.2020 CN 202010939250; 27.09.2020 CN 202011030862
(71) Applicant: Innovent Biologics (Suzhou) Co., Ltd., Suzhou, Jiangsu 215123 (CN)
(72) Inventor: ZOU, Jia, Suzhou, Jiangsu 215123 (CN); LI, Li, Suzhou, Jiangsu 215123 (CN); ZHOU, Shuaixiang, Suzhou, Jiangsu 215123 (CN); HE, Jianxing, Suzhou, Jiangsu 215123 (CN)
(74) Representative: Sagittarius IP
(86) International application number: PCT/CN2021/117348
(87) International publication number: WO 2022/052968

(57) **Abstract**

Provided are an antibody or an antigen-binding fragment for a coronavirus S protein, a nucleic acid encoding the antibody or the antigen-binding fragment, a host cell comprising the nucleic acid, and a method for preparing the antibody or the antigen-binding fragment. Also provided is a use of the antibody or the antigen-binding fragment in prevention, treatment, and/or diagnosis of coronavirus.

## Description

### TECHNICAL FIELD

The present invention relates generally to an antibody and use thereof. More specifically, the present invention relates to a monoclonal antibody and an antigen-binding fragment directed against a coronavirus spike protein, a method for preparing the same, and use thereof.

### BACKGROUND

Coronaviruses (CoVs) are single-stranded, positive-sense RNA (+ssRNA) viruses. The virions are spherical or ellipsoidal in shape and about 60-220 nm in diameter.

Among several human-pathogenic coronaviruses, most are associated with mild clinical symptoms (Su S, Wong G, Shi W et al., Epidemiology, genetic recombination, and pathogenesis of coronaviruses. Trends Microbiol 2016; 24: 490-502), but two coronaviruses pose a global threat to human health: one is severe acute respiratory syndromes coronavirus (SARS-CoV), which caused more than 8,000 human infections and 774 deaths in 37 countries and regions between 2002 and 2003 (Chan-Yeung M, Xu RH. SARS: epidemiology. *Respirology* 2003; 8 (suppl): S9-14); the other is 2019 novel coronavirus (2019-nCoV) responsible for novel coronavirus disease 2019 (COVID-19) in humans, which caused about 22.1 million human infections and about 780,000 deaths from December 2019 to August 2020.

The mortality rate of the coronavirus SARS-CoV is higher than that of 2019-nCoV, but the 2019-nCoV spreads very rapidly from human to human, so there is an urgent need for antibodies capable of neutralizing such coronavirus.

### SUMMARY

The present inventors have developed a group of antibodies capable of specifically binding to coronavirus spike proteins with high affinity, thereby inhibiting coronavirus infection in humans, thus meeting the above-mentioned need.

In a first aspect, the present invention provides an isolated anti-coronavirus S protein antibody or an antigen-binding fragment, comprising:
(a) 3 CDRs in an amino acid sequence of a heavy chain variable region set forth in SEQ ID NO: 2 and 3 CDRs in an amino acid sequence of a light chain variable region set forth in SEQ ID NO: 4; or a CDR variant having no more than 3 amino acid residue substitutions relative to any one of the 6 CDRs;
(b) 3 CDRs in an amino acid sequence of a heavy chain variable region set forth in SEQ ID NO: 6 and 3 CDRs in an amino acid sequence of a light chain variable region set forth in SEQ ID NO: 8; or a CDR variant having no more than 3 amino acid residue substitutions relative to any one of the 6 CDRs;
(c) 3 CDRs in an amino acid sequence of a heavy chain variable region set forth in SEQ ID NO: 10 and 3 CDRs in an amino acid sequence of a light chain variable region set forth in SEQ ID NO: 12; or a CDR variant having no more than 3 amino acid residue substitutions relative to any one of the 6 CDRs;
(d) 3 CDRs in an amino acid sequence of a heavy chain variable region set forth in SEQ ID NO: 14 and 3 CDRs in an amino acid sequence of a light chain variable region set forth in SEQ ID NO: 16; or a CDR variant having no more than 3 amino acid residue substitutions relative to any one of the 6 CDRs;
(e) 3 CDRs in an amino acid sequence of a heavy chain variable region set forth in SEQ ID NO: 18 and 3 CDRs in an amino acid sequence of a light chain variable region set forth in SEQ ID NO: 20; or a CDR variant having no more than 3 amino acid residue substitutions relative to any one of the 6 CDRs;
(f) 3 CDRs in an amino acid sequence of a heavy chain variable region set forth in SEQ ID NO: 22 and 3 CDRs in an amino acid sequence of a light chain variable region set forth in SEQ ID NO: 24; or a CDR variant having no more than 3 amino acid residue substitutions relative to any one of the 6 CDRs; or
(g) 3 CDRs in an amino acid sequence of a heavy chain variable region set forth in SEQ ID NO: 26 and 3 CDRs in an amino acid sequence of a light chain variable region set forth in SEQ ID NO: 28; or a CDR variant having no more than 3 amino acid residue substitutions relative to any one of the 6 CDRs.

In some embodiments, the present invention provides an isolated antibody or an antigen-binding fragment that specifically binds to a coronavirus S protein, comprising:
(a) an HCDR1 set forth in SEQ ID NO: 29 or a variant of the HCDR1 set forth in SEQ ID NO: 29 having no more than 3 amino acid residue substitutions, an HCDR2 set forth in SEQ ID NO: 30 or a variant of the HCDR2 set forth in SEQ ID NO: 30 having no more than 3 amino acid residue substitutions, and an HCDR3 set forth in SEQ ID NO: 31 or a variant of the HCDR3 set forth in SEQ ID NO: 31 having no more than 3 amino acid residue substitutions; and an LCDR1 set forth in SEQ ID NO: 32 or a variant of the LCDR1 set forth in SEQ ID NO: 32 having no more than 3 amino acid residue substitutions, an LCDR2 set forth in SEQ ID NO: 33 or a variant of the LCDR2 set forth in SEQ ID NO: 33 having no more than 3 amino acid residue substitutions, and an LCDR3 set forth in SEQ ID NO: 34 or a variant of the LCDR3 set forth in SEQ ID NO: 34 having no more than 3 amino acid residue substitutions;
(b) an HCDR1 set forth in SEQ ID NO: 35 or a variant of the HCDR1 set forth in SEQ ID NO: 35 having no more than 3 amino acid residue substitutions, an HCDR2 set forth in SEQ ID NO: 36 or a variant of the HCDR2 set forth in SEQ ID NO: 36 having no more than 3 amino acid residue substitutions, and an HCDR3 set forth in SEQ ID NO: 37 or a variant of the HCDR3 set forth in SEQ ID NO: 37 having no more than 3 amino acid residue substitutions; and an LCDR1 set forth in SEQ ID NO: 38 or a variant of the LCDR1 set forth in SEQ ID NO: 38 having no more than 3 amino acid residue substitutions, an LCDR2 set forth in SEQ ID NO: 39 or a variant of the LCDR2 set forth in SEQ ID NO: 39 having no more than 3 amino acid residue substitutions, and an LCDR3 set forth in SEQ ID NO: 40 or a variant of the LCDR3 set forth in SEQ ID NO: 40 having no more than 3 amino acid residue substitutions;
(c) an HCDR1 set forth in SEQ ID NO: 41 or a variant of the HCDR1 set forth in SEQ ID NO: 41 having no more than 3 amino acid residue substitutions, an HCDR2 set forth in SEQ ID NO: 42 or a variant of the HCDR2 set forth in SEQ ID NO: 42 having no more than 3 amino acid residue substitutions, and an HCDR3 set forth in SEQ ID NO: 43 or a variant of the HCDR3 set forth in SEQ ID NO: 43 having no more than 3 amino acid residue substitutions; and an LCDR1 set forth in SEQ ID NO: 44 or a variant of the LCDR1 set forth in SEQ ID NO: 44 having no more than 3 amino acid residue substitutions, an LCDR2 set forth in SEQ ID NO: 45 or a variant of the LCDR2 set forth in SEQ ID NO: 45 having no more than 3 amino acid residue substitutions, and an LCDR3 set forth in SEQ ID NO: 46 or a variant of the LCDR3 set forth in SEQ ID NO: 46 having no more than 3 amino acid residue substitutions;
(d) an HCDR1 set forth in SEQ ID NO: 47 or a variant of the HCDR1 set forth in SEQ ID NO: 47 having no more than 3 amino acid residue substitutions, an HCDR2 set forth in SEQ ID NO: 48 or a variant of the HCDR2 set forth in SEQ ID NO: 48 having no more than 3 amino acid residue substitutions, and an HCDR3 set forth in SEQ ID NO: 49 or a variant of the HCDR3 set forth in SEQ ID NO: 49 having no more than 3 amino acid residue substitutions; and an LCDR1 set forth in SEQ ID NO: 50 or a variant of the LCDR1 set forth in SEQ ID NO: 50 having no more than 3 amino acid residue substitutions, an LCDR2 set forth in SEQ ID NO: 51 or a variant of the LCDR2 set forth in SEQ ID NO: 51 having no more than 3 amino acid residue substitutions, and an LCDR3 set forth in SEQ ID NO: 52 or a variant of the LCDR3 set forth in SEQ ID NO: 52 having no more than 3 amino acid residue substitutions;
(e) an HCDR1 set forth in SEQ ID NO: 53 or a variant of the HCDR1 set forth in SEQ ID NO: 53 having no more than 3 amino acid residue substitutions, an HCDR2 set forth in SEQ ID NO: 54 or a variant of the HCDR2 set forth in SEQ ID NO: 54 having no more than 3 amino acid residue substitutions, and an HCDR3 set forth in SEQ ID NO: 55 or a variant of the HCDR3 set forth in SEQ ID NO: 55 having no more than 3 amino acid residue substitutions; and an LCDR1 set forth in SEQ ID NO: 56 or a variant of the LCDR1 set forth in SEQ ID NO: 56 having no more than 3 amino acid residue substitutions, an LCDR2 set forth in SEQ ID NO: 57 or a variant of the LCDR2 set forth in SEQ ID NO: 57 having no more than 3 amino acid residue substitutions, and an LCDR3 set forth in SEQ ID NO: 58 or a variant of the LCDR3 set forth in SEQ ID NO: 58 having no more than 3 amino acid residue substitutions;
(f) an HCDR1 set forth in SEQ ID NO: 59 or a variant of the HCDR1 set forth in SEQ ID NO: 59 having no more than 3 amino acid residue substitutions, an HCDR2 set forth in SEQ ID NO: 60 or a variant of the HCDR2 set forth in SEQ ID NO: 60 having no more than 3 amino acid residue substitutions, and an HCDR3 set forth in SEQ ID NO: 61 or a variant of the HCDR3 set forth in SEQ ID NO: 61 having no more than 3 amino acid residue substitutions; and an LCDR1 set forth in SEQ ID NO: 62 or a variant of the LCDR1 set forth in SEQ ID NO: 62 having no more than 3 amino acid residue substitutions, an LCDR2 set forth in SEQ ID NO: 63 or a variant of the LCDR2 set forth in SEQ ID NO: 63 having no more than 3 amino acid residue substitutions, and an LCDR3 set forth in SEQ ID NO: 64 or a variant of the LCDR3 set forth in SEQ ID NO: 64 having no more than 3 amino acid residue substitutions; or
(g) an HCDR1 set forth in SEQ ID NO: 65 or a variant of the HCDR1 set forth in SEQ ID NO: 65 having no more than 3 amino acid residue substitutions, an HCDR2 set forth in SEQ ID NO: 66 or a variant of the HCDR2 set forth in SEQ ID NO: 66 having no more than 3 amino acid residue substitutions, and an HCDR3 set forth in SEQ ID NO: 67 or a variant of the HCDR3 set forth in SEQ ID NO: 67 having no more than 3 amino acid residue substitutions; and an LCDR1 set forth in SEQ ID NO: 68 or a variant of the LCDR1 set forth in SEQ ID NO: 68 having no more than 3 amino acid residue substitutions, an LCDR2 set forth in SEQ ID NO: 69 or a variant of the LCDR2 set forth in SEQ ID NO: 69 having no more than 3 amino acid residue substitutions, and an LCDR3 set forth in SEQ ID NO: 70 or a variant of the LCDR3 set forth in SEQ ID NO: 70 having no more than 3 amino acid residue substitutions.

In some embodiments, the isolated antibody or the antigen-binding fragment of the present invention comprises:
(a) an HCDR1 set forth in SEQ ID NO: 29, an HCDR2 set forth in SEQ ID NO: 30, and an HCDR3 set forth in SEQ ID NO: 31; and an LCDR1 set forth in SEQ ID NO: 32, an LCDR2 set forth in SEQ ID NO: 33, and an LCDR3 set forth in SEQ ID NO: 34;
(b) an HCDR1 set forth in SEQ ID NO: 35, an HCDR2 set forth in SEQ ID NO: 36, and an HCDR3 set forth in SEQ ID NO: 37; and an LCDR1 set forth in SEQ ID NO: 38, an LCDR2 set forth in SEQ ID NO: 39, and an LCDR3 set forth in SEQ ID NO: 40;
(c) an HCDR1 set forth in SEQ ID NO: 41, an HCDR2 set forth in SEQ ID NO: 42, and an HCDR3 set forth in SEQ ID NO: 43; and an LCDR1 set forth in SEQ ID NO: 44, an LCDR2 set forth in SEQ ID NO: 45, and an LCDR3 set forth in SEQ ID NO: 46;
(d) an HCDR1 set forth in SEQ ID NO: 47, an HCDR2 set forth in SEQ ID NO: 48, and an HCDR3 set forth in SEQ ID NO: 49; and an LCDR1 set forth in SEQ ID NO: 50, an LCDR2 set forth in SEQ ID NO: 51, and an LCDR3 set forth in SEQ ID NO: 52;
(e) an HCDR1 set forth in SEQ ID NO: 53, an HCDR2 set forth in SEQ ID NO: 54, and an HCDR3 set forth in SEQ ID NO: 55; and an LCDR1 set forth in SEQ ID NO: 56, an LCDR2 set forth in SEQ ID NO: 57, and an LCDR3 set forth in SEQ ID NO: 58;
(f) an HCDR1 set forth in SEQ ID NO: 59, an HCDR2 set forth in SEQ ID NO: 60, and an HCDR3 set forth in SEQ ID NO: 61; and an LCDR1 set forth in SEQ ID NO: 62, an LCDR2 set forth in SEQ ID NO: 63, and an LCDR3 set forth in SEQ ID NO: 64; or
(g) an HCDR1 set forth in SEQ ID NO: 65, an HCDR2 set forth in SEQ ID NO: 66, and an HCDR3 set forth in SEQ ID NO: 67; and an LCDR1 set forth in SEQ ID NO: 68, an LCDR2 set forth in SEQ ID NO: 69, and an LCDR3 set forth in SEQ ID NO: 70.

In some embodiments, the isolated antibody or the antigen-binding fragment of the present invention comprises:
(a) a heavy chain variable region and a light chain variable region, wherein the heavy chain variable region comprises an amino acid sequence of SEQ ID NO: 2 or a sequence having at least 90%, 91%, 92%, 93%, 94%, 95%, 96%, 97%, 98%, or 99% identity thereto, and the light chain variable region comprises an amino acid sequence of SEQ ID NO: 4 or a sequence having at least 90%, 91%, 92%, 93%, 94%, 95%, 96%, 97%, 98%, or 99% identity thereto;
(b) a heavy chain variable region and a light chain variable region, wherein the heavy chain variable region comprises an amino acid sequence of SEQ ID NO: 6 or a sequence having at least 90%, 91%, 92%, 93%, 94%, 95%, 96%, 97%, 98%, or 99% identity thereto, and the light chain variable region comprises an amino acid sequence of SEQ ID NO: 8 or a sequence having at least 90%, 91%, 92%, 93%, 94%, 95%, 96%, 97%, 98%, or 99% identity thereto;
(c) a heavy chain variable region and a light chain variable region, wherein the heavy chain variable region comprises an amino acid sequence of SEQ ID NO: 10 or a sequence having at least 90%, 91%, 92%, 93%, 94%, 95%, 96%, 97%, 98%, or 99% identity thereto, and the light chain variable region comprises an amino acid sequence of SEQ ID NO: 12 or a sequence having at least 90%, 91%, 92%, 93%, 94%, 95%, 96%, 97%, 98%, or 99% identity thereto;
(d) a heavy chain variable region and a light chain variable region, wherein the heavy chain variable region comprises an amino acid sequence of SEQ ID NO: 14 or a sequence having at least 90%, 91%, 92%, 93%, 94%, 95%, 96%, 97%, 98%, or 99% identity thereto, and the light chain variable region comprises an amino acid sequence of SEQ ID NO: 16 or a sequence having at least 90%, 91%, 92%, 93%, 94%, 95%, 96%, 97%, 98%, or 99% identity thereto;
(e) a heavy chain variable region and a light chain variable region, wherein the heavy chain variable region comprises an amino acid sequence of SEQ ID NO: 18 or a sequence having at least 90%, 91%, 92%, 93%, 94%, 95%, 96%, 97%, 98%, or 99% identity thereto, and the light chain variable region comprises an amino acid sequence of SEQ ID NO: 20 or a sequence having at least 90%, 91%, 92%, 93%, 94%, 95%, 96%, 97%, 98%, or 99% identity thereto;
(f) a heavy chain variable region and a light chain variable region, wherein the heavy chain variable region comprises an amino acid sequence of SEQ ID NO: 22 or a sequence having at least 90%, 91%, 92%, 93%, 94%, 95%, 96%, 97%, 98%, or 99% identity thereto, and the light chain variable region comprises an amino acid sequence of SEQ ID NO: 24 or a sequence having at least 90%, 91%, 92%, 93%, 94%, 95%, 96%, 97%, 98%, or 99% identity thereto; or
(g) a heavy chain variable region and a light chain variable region, wherein the heavy chain variable region comprises an amino acid sequence of SEQ ID NO: 26 or a sequence having at least 90%, 91%, 92%, 93%, 94%, 95%, 96%, 97%, 98%, or 99% identity thereto, and the light chain variable region comprises an amino acid sequence of SEQ ID NO: 28 or a sequence having at least 90%, 91%, 92%, 93%, 94%, 95%, 96%, 97%, 98%, or 99% identity thereto.

In some embodiments, the isolated antibody or the antigen-binding fragment of the present invention is a fully human antibody or antigen-binding fragment.

In some embodiments, the isolated antibody or the antigen-binding fragment of the present invention is an IgG1, IgG2, IgG3, or IgG4 antibody; preferably, an IgG1 or IgG4 antibody; and more preferably, a human IgG1 or human IgG4 antibody. In some embodiments, the antigen-binding fragment of the present invention is an Fab, an Fab', an F(ab')₂, an Fv, a single-chain Fv, a single-chain Fab, or a diabody.

In some embodiments, the isolated anti-coronavirus S protein antibody or the antigen-binding fragment of the present invention is capable of specifically binding to an SARS-CoV-2 virus S protein and/or an SARS-COV virus S protein.

In some embodiments, the isolated anti-coronavirus S protein antibody or the antigen-binding fragment of the present invention is characterized by:
(a) binding to the coronavirus S protein with an EC₅₀ value of less than about 1 nM, preferably less than about 0.1 nM, and more preferably less than about 0.05 nM, as measured in an ELISA binding assay at 25 °C;
(b) blocking the binding of the coronavirus S protein to an isolated ACE2 protein with an IC₅₀ value of less than about 10 nM, preferably less than about 5 nM, and more preferably less than about 2 nM, as measured in an ELISA blocking assay at 25 °C;
(c) neutralizing a pseudotyped coronavirus with an IC₅₀ value of less than about 10 nM, preferably less than about 1 nM, and more preferably less than about 0.1 nM, as measured in a pseudotyped coronavirus neutralization assay; and
(d) neutralizing a true coronavirus with an EC₅₀ value of less than about 10 nM, preferably less than about 1 nM, and more preferably less than about 0.1 nM, as measured in a true coronavirus neutralization assay, whereby the true coronavirus is unable to cause a cytopathic effect.

In a second aspect, the present invention provides a nucleic acid encoding the antibody or the antigen-binding fragment described above in the first aspect, a vector (preferably, an expression vector) comprising the nucleic acid, and a host cell comprising the nucleic acid or the vector. In some embodiments, the host cell is prokaryotic or eukaryotic, e.g., selected from an *E*. *coli* cell, a yeast cell, a mammalian cell, and other cells suitable for preparing the antibody or the antigen-binding fragment. In some embodiments, the host cell is an HEK293 cell.

In a third aspect, the present invention provides a method for preparing the antibody or the antigen-binding fragment of the present invention, comprising culturing the host cell of the present invention under a condition suitable for expressing a nucleic acid encoding the antibody or the antigen-binding fragment of the present invention or encoding the present invention, and optionally isolating the antibody or the antigen-binding fragment of the present invention from the host cell or a culture medium.

In a fourth aspect, the present invention provides a pharmaceutical composition comprising the antibody or the antigen-binding fragment of the present invention and a pharmaceutically acceptable carrier. In some embodiments, the pharmaceutical composition comprises at least two antibodies or antigen-binding fragments of the present invention and a pharmaceutically acceptable carrier.

In a fifth aspect, the present invention provides use of the antibody or the antigen-binding fragment of the present invention in the preparation of a medicament for preventing and/or treating a coronavirus infection. In some embodiments, the coronavirus is SARS-CoV-2 virus and/or SARS-COV virus.

In a sixth aspect, the present invention provides a method for preventing and/or treating a coronavirus infection in a subject, comprising administering to the subject an effective amount of the antibody or the antigen-binding fragment of the present invention, or the pharmaceutical composition of the present invention. In some embodiments, the coronavirus is SARS-CoV-2 virus and/or SARS-COV virus.

The antibody of the present invention can effectively block and/or inhibit a coronavirus infection, and can be used in the prevention and/or treatment of coronavirus.

In a seventh aspect, the present invention provides a kit for detecting a coronavirus S protein in a sample, comprising the antibody or the antigen-binding fragment of the present invention for conducting the following steps:
(a) contacting the sample with the antibody or the antigen-binding fragment of the present invention; and
(b) detecting the formation of a complex by the antibody or the antigen-binding fragment of the present invention and the coronavirus S protein,
thereby determining the presence of a coronavirus infection in a sample from a subject or an individual. In one embodiment, the coronavirus is SARS-CoV-2 virus and/or SARS-COV virus.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIGs. 1, 2, 3, and 4 show the curves and EC₅₀ values for the binding of candidate antibody molecules to SARS-CoV-2 virus S proteins in an ELISA binding assay.
FIGs. 5, 6, 7, and 8 show the curves and IC₅₀ values for blocking the binding of SARS-CoV-2 virus S protein RBDs to isolated ACE2 proteins by candidate antibodies in an ELISA blocking assay.
FIG. 9 shows the curves and IC₅₀ values for the neutralization of pseudotyped SARS-CoV-2 virus by candidate antibodies in a pseudotyped coronavirus neutralization assay.
FIG. 10 shows the inhibitory effect of candidate antibodies on the cytopathic effect of SARS-CoV-2 euvirus.

The upper panel shows the inhibition of the candidate antibodies on the cytopathic effect of the SARS-CoV-2 euvirus, and the lower panel shows the cytopathic effect of the SARS-CoV-2 euvirus in the absence of the candidate antibodies.

FIG. 11 shows the curves and EC₅₀ values for the neutralization of SARS-CoV-2 euvirus by candidate antibodies and a combination of the candidate antibodies in an SARS-CoV-2 euvirus neutralization assay.

### DETAILED DESCRIPTION

Before the present invention is described in detail, it should be understood that the present invention is not limited to the particular methods or experimental conditions described herein since the methods and conditions may vary. Additionally, the terms used herein are for the purpose of describing particular embodiments only and are not intended to be limiting.

### I. Definitions

Unless otherwise defined, all technical and scientific terms used herein have the same meaning as those commonly understood by those of ordinary skill in the art. For the purposes of the present invention, the following terms are defined below.

The term "about" used in combination with a numerical value is intended to encompass the numerical values in a range from a lower limit less than the specified numerical value by 10% to an upper limit greater than the specified numerical value by 10%.

The term "and/or", when used to connect two or more options, should be understood to refer to any one of the options or any two or more of the options.

As used herein, the term "comprise" or "include" is intended to mean that the elements, integers or steps are included, but not to the exclusion of any other elements, integers or steps. The term "comprise" or "include" used herein, unless otherwise indicated, also encompasses the situation where the entirety consists of the described elements, integers or steps. For example, when referring to an antibody variable region "comprising" a particular sequence, it is also intended to encompass an antibody variable region consisting of the particular sequence.

The terms "2019-nCoV", "nCoV", "SARS-CoV-2", and "SARS-CoV2" are used interchangeably herein to refer to a coronavirus responsible for novel coronavirus disease 2019 (COVID-19) in humans, which has a strong ability to spread among humans.

The terms "SARS-CoV", "SARS-CoV-1", and "SARS-CoV1" are used interchangeably herein to refer to a coronavirus responsible for severe acute respiratory syndromes (SARS) in humans, which was endemic in 37 countries and regions between 2002 and 2003.

The term "antibody" is used herein in the broadest sense and includes, but is not limited to, monoclonal antibodies, polyclonal antibodies, and multispecific antibodies (such as bispecific antibodies), as long as they exhibit the desired antigen-binding activity. The antibody may be an intact antibody (e.g., having two full-length light chains and two full-length heavy chains) of any type and subtype (e.g., IgM, IgD, IgG1, IgG2, IgG3, IgG4, IgE, IgA1, and IgA2). A monomer of an intact antibody is a tetrapeptide chain molecule formed by connection between two full-length light chains and two full-length heavy chains by disulfide bonds, also known as a monomer of an Ig molecule. Antibody monomers are the basic structures that constitute an antibody.

"Monoclonal antibody" is an antibody produced by a single clone of B lymphocytes or by cells into which the light and heavy chain genes of a single antibody have been transfected. The monoclonal antibodies are prepared by methods known to those skilled in the art.

As used herein, "isolated antibody" is intended to refer to an antibody that is substantially free of other antibodies (Abs) having different antigenic specificities (e.g., an isolated antibody or an antigen-binding fragment thereof that specifically binds to a coronavirus S protein is substantially free of Abs that specifically bind to antigens other than the coronavirus S protein).

As used herein, "blocking antibody", "neutralization antibody", "antibody having neutralizing activity", and "neutralizing antibody" are used interchangeably herein to refer to an antibody that binds to or interacts with a target antigen and prevents the target antigen from binding to or associating with a binding ligand, such as a receptor, thereby inhibiting or blocking a biological response that would otherwise occur due to the interaction between the target antigen and the binding ligand, such as the receptor. In the context of the present invention, it means that the binding of the antibody to the coronavirus S protein results in the inhibition of at least one biological activity of the coronavirus. For example, the neutralizing antibody of the present invention may prevent or block the binding of the coronavirus S protein to ACE2.

"Epitope" refers to an antigenic determinant that interacts with a specific antigen-binding site, called a paratope, in the variable region of an antibody molecule. A single antigen may have more than one epitope. Thus, different antibodies may bind to different regions on the antigen and may have different biological effects. The epitope can be formed from contiguous amino acids, or non-contiguous amino acids juxtaposed via tertiary folding of a protein. Epitopes formed from contiguous amino acids are generally retained when exposed to a denaturing solvent, while epitopes formed by tertiary folding are generally lost when treated with the denaturing solvent. Epitopes generally comprise at least 3, and more generally at least 5, about 9, or about 8-10 amino acids in a unique spatial conformation.

The terms "whole antibody", "full-length antibody", "complete antibody" and "intact antibody" are used interchangeably herein to refer to a glycoprotein comprising at least two heavy chains (HC) and two light chains (LC) interconnected by disulfide bonds. Each heavy chain consists of a heavy chain variable region (abbreviated herein as VH) and a heavy chain constant region. Each heavy chain constant region consists of 3 domains CH1, CH2 and CH3. Each light chain consists of a light chain variable region (abbreviated herein as VL) and a light chain constant region. Each light chain constant region consists of one domain CL. Mammalian heavy chains are classified as α, δ, ε, γ, and µ. Mammalian light chains are classified as either λ or κ. Immunoglobulins comprising α, δ, ε, γ, and µ heavy chains are classified as immunoglobulins IgA, IgD, IgE, IgG, and IgM, respectively.

The light chain variable region and the heavy chain variable region each comprise a "framework" region interspersed with three highly variable regions (also called "complementarity determining regions" or "CDRs"). "Complementarity determining region" or "CDR region" or "CDR" or "highly variable region" (used interchangeably herein with hypervariable region "HVR"), is a region in an antibody variable domain that is highly variable in sequence and forms a structurally defined loop ("hypervariable loop") and/or comprises antigen-contacting residues ("antigen-contacting sites"). CDRs are primarily responsible for binding to antigen epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2 and HCDR3, whereas the CDRs located in the light chain variable domain of the antibody are referred to as LCDR1, LCDR2 and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature, 342:877-883; Al-Lazikani et al., Standard conformations for the canonical structures of immunoglobulins, Journal of Molecular Biology, 273:927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

However, it should be noted that boundaries of CDRs of variable regions of the same antibody based on different assignment systems may differ. That is, the CDR sequences of variable regions of the same antibody defined by different assignment systems differ. For example, the residue ranges defined by different assignment systems for Kabat- and Chothia-numbered CDR regions are shown in table A below.

**Table A. CDR residue ranges defined by different assignment systems**

| Loop | Kabat CDR | AbM | Chothia | Contact | IMGT |
|---|---|---|---|---|---|
| L1 | L24-L34 | L24-L34 | L24-L34 | L30-L36 | L27-L32 |
| L2 | L50-L56 | L50-L56 | L50-L56 | L46-L55 | L50-L52 |
| L3 | L89-L97 | L89-L97 | L89-L97 | L89-L96 | L89-L96 |
| H1 | H31-H35b Kabat numbering | H26-H35b | H26-H32...34 | H30-H35b | H26-H35b |
| H1 | H31-H35 Chothia numbering | H26-H35 | H26-H32 | H30-H35 | H26-H35 |
| H2 | H50-H65 | H50-H58 | H52-H56 | H47-H58 | H51-H57 |
| H3 | H95-H102 | H95-H102 | H95-H102 | H93-H101 | H93-H102 |

Accordingly, when it comes to defining an antibody with specific CDR sequences defined in the present invention, the scope of antibody also encompasses such antibodies whose variable region sequences comprise the specific CDR sequences, but have claimed CDR boundaries different from the specific CDR boundaries defined by the present invention due to a different scheme (e.g., different assignment system rules or their combinations) applied.

The boundaries of the CDRs of the antibodies of the present invention can be determined based on human evaluation according to any scheme in the art or a combination thereof. Unless otherwise stated, the term "CDR" or "CDR sequence" used herein encompasses CDR sequences determined by any one of the schemes above.

The sequences of the framework regions of the different light or heavy chains are relatively conserved within a species (e.g., human). The framework regions of the antibody, which are the combined framework regions of the component light and heavy chains, are used to locate and align the CDRs in three-dimensional space. The CDRs are primarily responsible for binding to antigen epitopes. Antibodies with different specificities (i.e., different binding sites for different antigens) have different CDRs. Although the CDRs differ from antibody to antibody, only a limited number of amino acid positions within the CDRs are directly involved in antigen binding. These positions within the CDRs are called specificity-determining residues (SDRs).

The term "antigen-binding fragment" is a portion or segment of an intact or a complete antibody that has fewer amino acid residues than an intact or a complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, an Fab, an Fab', an F(ab')₂, an Fv, a single-chain Fv (scFv), a single-chain Fab, a diabody, a single-domain antibody (sdAb, nanobody), a camel Ig, an Ig NAR, an F(ab)'₃ fragment, a bis-scFv, an (scFv)₂, a minibody, a bifunctional antibody, a trifunctional antibody, a tetrafunctional antibody, and a disulfide-stabilized Fv protein ("dsFv"). The term also includes genetically engineered forms, such as a chimeric antibody (e.g., a humanized mouse antibody), a heteroconjugate antibodiy (e.g., a bispecific antibody), and an antigen-binding fragment thereof. For a more detailed description, see also: Pierce Catalog and Handbook, 1994-1995 (PierceChemical Co., Rockford, IL); and Kuby, Immunology, 3rd Ed., W.H.Freeman&Co., New York, 1997.

An Fab fragment includes a heavy chain variable domain and a light chain variable domain, and also includes the constant domain of the light chain and the first constant domain (CH1) of the heavy chain. Fab' fragments differ from Fab fragments by the addition of several residues to the carboxyl terminal of the heavy chain CH1 domain, including one or more cysteines from an antibody hinge region. Fab'-SH is the designation used herein for Fab', wherein the cysteine residue of the constant domain carries a free thiol group. F(ab')₂ antibody fragments were originally generated as pairs of Fab' fragments with hinge cysteines between them. Other chemical couplings of the antibody fragments are also known.

"Fv" is the smallest antibody fragment that comprises a complete antigen-binding site. In one embodiment, a double-chain Fv consists of one heavy chain variable domain and one light chain variable domain in a tight, non-covalently associated dimer. In a single-chain Fv(scFv), one heavy chain variable domain and one light chain variable domain can be covalently linked via a flexible peptide linker so that the light chain and heavy chain can be associated with a structure similar to the "dimer" structure of the double-chain type. In this configuration, the three highly variable regions (HVRs) of each variable domain interact to define the antigen-binding site on the surface of the VH-VL dimer. The six HVRs collectively confer antigen-binding specificity to the antibody. However, even a single variable domain (or half of an Fv comprising only three HVRs specific for an antigen) has the ability to recognize and bind to an antigen, but with a lower affinity than the intact binding site.

The term "specifically bind" or "bind", when used in reference to an antigen and an antibody, means that the antibody forms a complex with the antigen which is relatively stable under physiological conditions. Methods for determining whether an antibody specifically binds to an antigen are well known in the art.

"Affinity" refers to the strength of the sum of all non-covalent interactions between a single binding site of a molecule (such as an antibody) and its binding ligand (such as an antigen). Unless otherwise stated, when used herein, "binding affinity" refers to the intrinsic binding affinity that reflects a 1:1 interaction between members of a bound pair (such as an antibody and an antigen). The affinity of a molecule X for its ligand Y can generally be represented by the binding equilibrium dissociation constant (K_{D}). Affinity can be measured by common methods known in the art, including those known in the prior art and described herein.

As used herein, the term "variant" refers to a heavy chain variable region or a light chain variable region that has been modified by at least one, e.g., 1, 2, or 3 amino acid substitutions, deletions, or additions, wherein the modified antigen-binding protein comprising the heavy chain or light chain variant substantially retains the biological characteristics of the antigen-binding protein prior to modification. In one embodiment, an antigen-binding protein comprising a variant heavy chain variable region or light chain variable region sequence retains 60%, 70%, 80%, 90%, or 100% of the biological characteristics of the antigen-binding protein prior to modification. It should be understood that each heavy chain variable region or light chain variable region may be modified individually or in combination with another heavy chain variable region or light chain variable region. The antigen-binding protein of the present disclosure comprises an amino acid sequence of a heavy chain variable region having 90%, 91%, 92%, 93%, 94%, 95%, 96%, 97%, 98%, or 99% homology to the amino acid sequence of the heavy chain variable region described herein. The antigen-binding protein of the present disclosure comprises an amino acid sequence of a light chain variable region having 90%, 91%, 92%, 93%, 94%, 95%, 96%, 97%, 98%, or 99% homology to the amino acid sequence of the light chain variable region described herein. The percent homology may be achieved throughout the entire heavy chain variable region and/or the entire light chain variable region, or the percent homology may be limited to the framework region, while the sequences corresponding to the CDRs have 100% identity to the CDRs disclosed herein within the heavy chain variable region and/or the light chain variable region. As used herein, the term "CDR variant" refers to a CDR that has been modified by at least one, e.g., 1, 2, or 3 amino acid substitutions, deletions, or additions, wherein the modified antigen-binding protein comprising the CDR variant substantially retains the biological characteristics of the antigen-binding protein prior to modification. In one embodiment, an antigen-binding protein comprising a variant CDR retains 60%, 70%, 80%, 90%, or 100% of the biological characteristics of the antigen-binding protein prior to modification. It should be understood that each CDR that can be modified may be modified individually or in combination with another CDR. In one embodiment, the modification is a substitution, particularly a conservative substitution.

As is known in the art, "polynucleotide" or "nucleic acid" as used interchangeably herein refers to a chain of nucleotides of any length, and includes DNA and RNA. The nucleotides may be deoxyribonucleotides, ribonucleotides, modified nucleotides or bases, and/or analogs thereof, or any substrate capable of being incorporated into a strand by a DNA or RNA polymerase.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossom 62 scoring matrix with a gap penalty of 12, a gap extension penalty of 4, and a frameshift gap penalty of 5.

The percent identity between two amino acid sequences or nucleotide sequences can also be determined with PAM120 weighted remainder table, a gap length penalty of 12 and a gap penalty of 4, using the E. Meyers and W. Miller algorithm ((1989) CABIOS, 4:11-17) which has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

The terms "host cell", "host cell line", and "host cell culture" are used interchangeably herein and refer to cells into which exogenous nucleic acids have been introduced, including progenies of such cells. Host cells include "transformants" and "transformed cells", which include primary transformed cells and progenies derived therefrom, regardless of the number of passages. Progenies may not be exactly the same as parent cells in terms of nucleic acid content, but may contain mutations. Mutant progenies having the same function or biological activity as cells that are screened or selected from the initially transformed cells are included herein. As used herein, "subject" or "individual" refers to an animal, preferably a mammal, and more preferably a human, in need of amelioration, prevention, and/or treatment of a disease or disorder, such as a viral infection. Mammal also includes, but is not limited to, farm animals, racing animals, pets, primates, horses, dogs, cats, mice, and rats. The term includes human subjects suffering from or at risk of suffering from a coronavirus infection. In the present invention, administering the antibody described herein or the pharmaceutical composition or product described herein to a subject in need thereof means administering an effective amount of the antibody or the pharmaceutical composition or product, etc.

As used herein, the term "effective amount" means an amount of a drug or pharmaceutical preparation that elicits the biological or medicinal response in a tissue, system, animal or human that is being sought, for example, by a researcher or clinician. Furthermore, the term "therapeutically effective amount" means an amount that causes improved treatment, cure, prevention, or alleviation of a disease, disorder or side effect, or an amount that causes a reduction in the rate of progression of a disease or condition, as compared to a corresponding subject that does not receive that amount. The term also includes within its scope an amount effective to enhance normal physiological function.

### II. Coronavirus against which the antibody of the present invention is directed

Coronavirus (including SARS-CoV and 2019-nCoV) is an enveloped virus with a viral structure mainly formed by viral structural proteins (e.g., spike (S) protein, membrane (M) protein, envelope (E) protein, and nucleocapsid (N) protein), wherein the S protein, the M protein, and the E protein are all embedded in the viral envelope, and the N protein interacts with viral RNA and is located at the core of the virion to form a nucleocapsid (Fehr, A. R. et al., Coronaviruses: An overview of their replication and pathogenesis. Methods Mol.Biol. 2015, 1282, 1-23).

The S protein is a highly glycosylated protein that forms a homotrimeric spike on the surface of the virion, mediates viral invasion by binding to a host cell receptor, and determines the host specificity of the virus. After sequence alignment, the S protein of 2019-nCoV virus was found to have 75% similarity to that of SARS-CoV virus. Both SARS-CoV and 2019-nCoV infect the host by the binding of a receptor binding domain (RBD) in the S protein to the ACE2 receptor expressed on the surface of the host cell. High affinity neutralizing antibodies that are directed against the S proteins of SARS-CoV and 2019-nCoV and block the binding of the S proteins to the ACE2 receptors are expected to be effective in preventing and treating the coronavirus infection.

### III. Antibody of the present invention directed against coronavirus S protein

The terms "antibody directed against coronavirus S protein", "anti-coronavirus S protein antibody", "anti-S protein antibody", "coronavirus S protein antibody", "S protein antibody", and "antibody that binds to S protein" are used interchangeably herein to refer to an antibody of the present invention that is capable of binding to a coronavirus S protein (e.g., 2019-nCoV S protein, SARS-CoV S protein) with sufficient affinity such that the antibody can be used as a diagnostic, prophylactic, and/or therapeutic agent targeting the coronavirus S protein.

The antibody and the antigen-binding fragment of the present invention specifically bind to a coronavirus S protein with high affinity. In some embodiments, the antibody of the present invention is a blocking antibody or a neutralizing antibody, wherein the antibody can bind to a coronavirus S protein and block the binding of the coronavirus S protein to ACE2. In some embodiments, the blocking antibody or the neutralizing antibody can be used to prevent a coronavirus infection and/or treat an individual with the coronavirus infection.

In some embodiments, the coronavirus S protein antibody of the present invention specifically binds to a coronavirus S protein, comprising:
(a) 3 CDRs in an amino acid sequence of a heavy chain variable region set forth in SEQ ID NO: 2 and 3 CDRs in an amino acid sequence of a light chain variable region set forth in SEQ ID NO: 4; or a CDR variant having no more than 3 amino acid residue substitutions relative to any one of the 6 CDRs;
(b) 3 CDRs in an amino acid sequence of a heavy chain variable region set forth in SEQ ID NO: 6 and 3 CDRs in an amino acid sequence of a light chain variable region set forth in SEQ ID NO: 8; or a CDR variant having no more than 3 amino acid residue substitutions relative to any one of the 6 CDRs;
(c) 3 CDRs in an amino acid sequence of a heavy chain variable region set forth in SEQ ID NO: 10 and 3 CDRs in an amino acid sequence of a light chain variable region set forth in SEQ ID NO: 12; or a CDR variant having no more than 3 amino acid residue substitutions relative to any one of the 6 CDRs;
(d) 3 CDRs in an amino acid sequence of a heavy chain variable region set forth in SEQ ID NO: 14 and 3 CDRs in an amino acid sequence of a light chain variable region set forth in SEQ ID NO: 16; or a CDR variant having no more than 3 amino acid residue substitutions relative to any one of the 6 CDRs;
(e) 3 CDRs in an amino acid sequence of a heavy chain variable region set forth in SEQ ID NO: 18 and 3 CDRs in an amino acid sequence of a light chain variable region set forth in SEQ ID NO: 20; or a CDR variant having no more than 3 amino acid residue substitutions relative to any one of the 6 CDRs;
(f) 3 CDRs in an amino acid sequence of a heavy chain variable region set forth in SEQ ID NO: 22 and 3 CDRs in an amino acid sequence of a light chain variable region set forth in SEQ ID NO: 24; or a CDR variant having no more than 3 amino acid residue substitutions relative to any one of the 6 CDRs; or
(g) 3 CDRs in an amino acid sequence of a heavy chain variable region set forth in SEQ ID NO: 26 and 3 CDRs in an amino acid sequence of a light chain variable region set forth in SEQ ID NO: 28; or a CDR variant having no more than 3 amino acid residue substitutions relative to any one of the 6 CDRs.

In some embodiments, the coronavirus S protein antibody of the present invention binds to a mammalian coronavirus S protein, e.g., a human coronavirus S protein or a monkey coronavirus S protein. For example, the coronavirus S protein antibody of the present invention specifically binds to an epitope (e.g., a linear or conformational epitope) on the coronavirus S protein.

In some embodiments, the coronavirus S protein antibody of the present invention has one or more of the following properties:
(a) binding to the coronavirus S protein with an EC₅₀ value of less than about 1 nM, preferably less than about 0.1 nM, and more preferably less than about 0.05 nM, as measured in an ELISA binding assay at 25 °C;
(b) blocking the binding of the coronavirus S protein to an isolated ACE2 protein with an IC₅₀ value of less than about 10 nM, preferably less than about 5 nM, and more preferably less than about 2 nM, as measured in an ELISA blocking assay at 25 °C;
(c) neutralizing a pseudotyped coronavirus with an IC₅₀ value of less than about 10 nM, preferably less than about 1 nM, and more preferably less than about 0.1 nM, as measured in a pseudotyped coronavirus neutralization assay; and
(d) neutralizing a true coronavirus with an EC₅₀ value of less than about 10 nM, preferably less than about 1 nM, and more preferably less than about 0.1 nM, as measured in a true coronavirus neutralization assay, whereby the true coronavirus is unable to cause a cytopathic effect.

In some embodiments, the coronavirus S protein antibody of the present invention comprises:
(a) an HCDR1 set forth in SEQ ID NO: 29 or a variant of the HCDR1 set forth in SEQ ID NO: 29 having no more than 3 amino acid residue substitutions, an HCDR2 set forth in SEQ ID NO: 30 or a variant of the HCDR2 set forth in SEQ ID NO: 30 having no more than 3 amino acid residue substitutions, and an HCDR3 set forth in SEQ ID NO: 31 or a variant of the HCDR3 set forth in SEQ ID NO: 31 having no more than 3 amino acid residue substitutions; and an LCDR1 set forth in SEQ ID NO: 32 or a variant of the LCDR1 set forth in SEQ ID NO: 32 having no more than 3 amino acid residue substitutions, an LCDR2 set forth in SEQ ID NO: 33 or a variant of the LCDR2 set forth in SEQ ID NO: 33 having no more than 3 amino acid residue substitutions, and an LCDR3 set forth in SEQ ID NO: 34 or a variant of the LCDR3 set forth in SEQ ID NO: 34 having no more than 3 amino acid residue substitutions;
(b) an HCDR1 set forth in SEQ ID NO: 35 or a variant of the HCDR1 set forth in SEQ ID NO: 35 having no more than 3 amino acid residue substitutions, an HCDR2 set forth in SEQ ID NO: 36 or a variant of the HCDR2 set forth in SEQ ID NO: 36 having no more than 3 amino acid residue substitutions, and an HCDR3 set forth in SEQ ID NO: 37 or a variant of the HCDR3 set forth in SEQ ID NO: 37 having no more than 3 amino acid residue substitutions; and an LCDR1 set forth in SEQ ID NO: 38 or a variant of the LCDR1 set forth in SEQ ID NO: 38 having no more than 3 amino acid residue substitutions, an LCDR2 set forth in SEQ ID NO: 39 or a variant of the LCDR2 set forth in SEQ ID NO: 39 having no more than 3 amino acid residue substitutions, and an LCDR3 set forth in SEQ ID NO: 40 or a variant of the LCDR3 set forth in SEQ ID NO: 40 having no more than 3 amino acid residue substitutions;
(c) an HCDR1 set forth in SEQ ID NO: 41 or a variant of the HCDR1 set forth in SEQ ID NO: 41 having no more than 3 amino acid residue substitutions, an HCDR2 set forth in SEQ ID NO: 42 or a variant of the HCDR2 set forth in SEQ ID NO: 42 having no more than 3 amino acid residue substitutions, and an HCDR3 set forth in SEQ ID NO: 43 or a variant of the HCDR3 set forth in SEQ ID NO: 43 having no more than 3 amino acid residue substitutions; and an LCDR1 set forth in SEQ ID NO: 44 or a variant of the LCDR1 set forth in SEQ ID NO: 44 having no more than 3 amino acid residue substitutions, an LCDR2 set forth in SEQ ID NO: 45 or a variant of the LCDR2 set forth in SEQ ID NO: 45 having no more than 3 amino acid residue substitutions, and an LCDR3 set forth in SEQ ID NO: 46 or a variant of the LCDR3 set forth in SEQ ID NO: 46 having no more than 3 amino acid residue substitutions;
(d) an HCDR1 set forth in SEQ ID NO: 47 or a variant of the HCDR1 set forth in SEQ ID NO: 47 having no more than 3 amino acid residue substitutions, an HCDR2 set forth in SEQ ID NO: 48 or a variant of the HCDR2 set forth in SEQ ID NO: 48 having no more than 3 amino acid residue substitutions, and an HCDR3 set forth in SEQ ID NO: 49 or a variant of the HCDR3 set forth in SEQ ID NO: 49 having no more than 3 amino acid residue substitutions; and an LCDR1 set forth in SEQ ID NO: 50 or a variant of the LCDR1 set forth in SEQ ID NO: 50 having no more than 3 amino acid residue substitutions, an LCDR2 set forth in SEQ ID NO: 51 or a variant of the LCDR2 set forth in SEQ ID NO: 51 having no more than 3 amino acid residue substitutions, and an LCDR3 set forth in SEQ ID NO: 52 or a variant of the LCDR3 set forth in SEQ ID NO: 52 having no more than 3 amino acid residue substitutions;
(e) an HCDR1 set forth in SEQ ID NO: 53 or a variant of the HCDR1 set forth in SEQ ID NO: 53 having no more than 3 amino acid residue substitutions, an HCDR2 set forth in SEQ ID NO: 54 or a variant of the HCDR2 set forth in SEQ ID NO: 54 having no more than 3 amino acid residue substitutions, and an HCDR3 set forth in SEQ ID NO: 55 or a variant of the HCDR3 set forth in SEQ ID NO: 55 having no more than 3 amino acid residue substitutions; and an LCDR1 set forth in SEQ ID NO: 56 or a variant of the LCDR1 set forth in SEQ ID NO: 56 having no more than 3 amino acid residue substitutions, an LCDR2 set forth in SEQ ID NO: 57 or a variant of the LCDR2 set forth in SEQ ID NO: 57 having no more than 3 amino acid residue substitutions, and an LCDR3 set forth in SEQ ID NO: 58 or a variant of the LCDR3 set forth in SEQ ID NO: 58 having no more than 3 amino acid residue substitutions;
(f) an HCDR1 set forth in SEQ ID NO: 59 or a variant of the HCDR1 set forth in SEQ ID NO: 59 having no more than 3 amino acid residue substitutions, an HCDR2 set forth in SEQ ID NO: 60 or a variant of the HCDR2 set forth in SEQ ID NO: 60 having no more than 3 amino acid residue substitutions, and an HCDR3 set forth in SEQ ID NO: 61 or a variant of the HCDR3 set forth in SEQ ID NO: 61 having no more than 3 amino acid residue substitutions; and an LCDR1 set forth in SEQ ID NO: 62 or a variant of the LCDR1 set forth in SEQ ID NO: 62 having no more than 3 amino acid residue substitutions, an LCDR2 set forth in SEQ ID NO: 63 or a variant of the LCDR2 set forth in SEQ ID NO: 63 having no more than 3 amino acid residue substitutions, and an LCDR3 set forth in SEQ ID NO: 64 or a variant of the LCDR3 set forth in SEQ ID NO: 64 having no more than 3 amino acid residue substitutions; or
(g) an HCDR1 set forth in SEQ ID NO: 65 or a variant of the HCDR1 set forth in SEQ ID NO: 65 having no more than 3 amino acid residue substitutions, an HCDR2 set forth in SEQ ID NO: 66 or a variant of the HCDR2 set forth in SEQ ID NO: 66 having no more than 3 amino acid residue substitutions, and an HCDR3 set forth in SEQ ID NO: 67 or a variant of the HCDR3 set forth in SEQ ID NO: 67 having no more than 3 amino acid residue substitutions; and an LCDR1 set forth in SEQ ID NO: 68 or a variant of the LCDR1 set forth in SEQ ID NO: 68 having no more than 3 amino acid residue substitutions, an LCDR2 set forth in SEQ ID NO: 69 or a variant of the LCDR2 set forth in SEQ ID NO: 69 having no more than 3 amino acid residue substitutions, and an LCDR3 set forth in SEQ ID NO: 70 or a variant of the LCDR3 set forth in SEQ ID NO: 70 having no more than 3 amino acid residue substitutions.

In some embodiments, the amino acid residue substitutions in the CDRs are conservative amino acid residue substitutions.

In some embodiments, the coronavirus S protein antibody or the antigen-binding fragment of the present invention comprises:
(a) a heavy chain variable region and a light chain variable region, wherein the heavy chain variable region comprises an amino acid sequence of SEQ ID NO: 2 or a sequence having at least 90%, 91%, 92%, 93%, 94%, 95%, 96%, 97%, 98%, or 99% identity thereto, and the light chain variable region comprises an amino acid sequence of SEQ ID NO: 4 or a sequence having at least 90%, 91%, 92%, 93%, 94%, 95%, 96%, 97%, 98%, or 99% identity thereto;
(b) a heavy chain variable region and a light chain variable region, wherein the heavy chain variable region comprises an amino acid sequence of SEQ ID NO: 6 or a sequence having at least 90%, 91%, 92%, 93%, 94%, 95%, 96%, 97%, 98%, or 99% identity thereto, and the light chain variable region comprises an amino acid sequence of SEQ ID NO: 8 or a sequence having at least 90%, 91%, 92%, 93%, 94%, 95%, 96%, 97%, 98%, or 99% identity thereto;
(c) a heavy chain variable region and a light chain variable region, wherein the heavy chain variable region comprises an amino acid sequence of SEQ ID NO: 10 or a sequence having at least 90%, 91%, 92%, 93%, 94%, 95%, 96%, 97%, 98%, or 99% identity thereto, and the light chain variable region comprises an amino acid sequence of SEQ ID NO: 12 or a sequence having at least 90%, 91%, 92%, 93%, 94%, 95%, 96%, 97%, 98%, or 99% identity thereto;
(d) a heavy chain variable region and a light chain variable region, wherein the heavy chain variable region comprises an amino acid sequence of SEQ ID NO: 14 or a sequence having at least 90%, 91%, 92%, 93%, 94%, 95%, 96%, 97%, 98%, or 99% identity thereto, and the light chain variable region comprises an amino acid sequence of SEQ ID NO: 16 or a sequence having at least 90%, 91%, 92%, 93%, 94%, 95%, 96%, 97%, 98%, or 99% identity thereto;
(e) a heavy chain variable region and a light chain variable region, wherein the heavy chain variable region comprises an amino acid sequence of SEQ ID NO: 18 or a sequence having at least 90%, 91%, 92%, 93%, 94%, 95%, 96%, 97%, 98%, or 99% identity thereto, and the light chain variable region comprises an amino acid sequence of SEQ ID NO: 20 or a sequence having at least 90%, 91%, 92%, 93%, 94%, 95%, 96%, 97%, 98%, or 99% identity thereto;
(f) a heavy chain variable region and a light chain variable region, wherein the heavy chain variable region comprises an amino acid sequence of SEQ ID NO: 22 or a sequence having at least 90%, 91%, 92%, 93%, 94%, 95%, 96%, 97%, 98%, or 99% identity thereto, and the light chain variable region comprises an amino acid sequence of SEQ ID NO: 24 or a sequence having at least 90%, 91%, 92%, 93%, 94%, 95%, 96%, 97%, 98%, or 99% identity thereto; or
(g) a heavy chain variable region and a light chain variable region, wherein the heavy chain variable region comprises an amino acid sequence of SEQ ID NO: 26 or a sequence having at least 90%, 91%, 92%, 93%, 94%, 95%, 96%, 97%, 98%, or 99% identity thereto, and the light chain variable region comprises an amino acid sequence of SEQ ID NO: 28 or a sequence having at least 90%, 91%, 92%, 93%, 94%, 95%, 96%, 97%, 98%, or 99% identity thereto.

In some embodiments, in a sequence having at least 90%, 91%, 92%, 93%, 94%, 95%, 96%, 97%, 98%, or 99% identity to heavy and light chain variable regions of specific sequence numbers, an amino acid alteration thereof as compared to the heavy and light chain variable regions of specific sequence numbers does not occur in the CDR region.

In some embodiments, the present invention relates to the coronavirus S protein antibody or the antigen-binding fragment described above, wherein the antibody comprises a light chain constant region and/or a heavy chain constant region of human or primate origin.

In some embodiments, the coronavirus S protein antibody of the present invention is an IgG class antibody, particularly, an IgG1, IgG2, IgG3, or IgG4 antibody, preferably, an IgG1 or IgG4 antibody, and more preferably, a human IgG1 or human IgG4 antibody.

In some embodiments of the present invention, the amino acid alteration described herein includes amino acid substitution, insertion or deletion. Preferably, the amino acid alteration described herein is an amino acid substitution, preferably a conservative substitution. The "conservative substitution" refers to a substitution of an amino acid by another amino acid of the same class, for example, the substitution of an acidic amino acid by another acidic amino acid, the substitution of a basic amino acid by another basic amino acid, or the substitution of a neutral amino acid by another neutral amino acid. Exemplary substitutions are shown in Table B below:

**Table B. Exemplary amino acid substitutions**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In a preferred embodiment, the amino acid alteration described herein occurs in a region outside the CDR (e.g., in FR). More preferably, the amino acid alteration described herein occurs in the Fc region. In some embodiments, provided is an anti-coronavirus S protein antibody comprising an Fc domain containing one or more mutations. In one embodiment, the Fc region of the anti-coronavirus S protein antibody is the entire portion of a human constant region. The Fc region is directly involved in complement activation, C1q binding, C3 activation, and Fc receptor binding. The binding sites in the Fc region cause binding to C1q. Such binding sites are known in the art and are described, for example, by Thommesen, J. E. et al., Mol. Immunol. 37 (2000) 995-1004; Idusogie, E. E. et al., J. Immunol. 164 (2000) 4178-4184; and Hezareh, M. et al., J. Virol. 75 (2001) 12161-12168. Such binding sites are, for example, L234, L235, D270, N297, E318, K320, K322, P331, and P329 (numbered according to the EU index of Kabat). Antibody subclasses IgG1, IgG2, and IgG3 are generally shown to activate complement, bind to C1q, and activate C3, while IgG4 does not activate the complement system, does not bind to C1q, and does not activate C3. In one embodiment, the Fc region is a human IgG4 subclass comprising mutations S228P and/or L235E and/or P329G (numbered according to the EU index of Kabat). In one embodiment, the Fc region is a human IgG1 subclass comprising mutations L234A and L235A, and optionally P329G (numbered according to the EU index of Kabat).

In one embodiment, the present invention includes an anti-coronavirus S protein antibody comprising an Fc domain, wherein the Fc domain comprises an S108P mutation in an IgG4 hinge region to facilitate dimer stabilization. Any possible combination of the aforementioned Fc domain mutations and other mutations within the variable domains of the antibodies disclosed herein are included within the scope of the present invention.

In certain embodiments, the coronavirus S protein antibody provided herein is altered to increase or decrease the extent to which it is glycosylated. Addition or deletion of glycosylation sites of the coronavirus S protein antibody can be conveniently achieved by altering the amino acid sequence to create or remove one or more glycosylation sites.

When the coronavirus S protein antibody comprises an Fc region, the carbohydrates attached to the Fc region may be altered. In some applications, modifications that remove undesired glycosylation sites may be useful, for example, removing fucose modules to enhance antibody-dependent cellular cytotoxicity (ADCC) (see Shield et al., (2002) JBC277:26733). In other applications, galactosylation modification can be carried out to modulate complement-dependent cytotoxicity (CDC).

### IV. Nucleic acid of the present invention and host cell comprising same

In one aspect, the present invention provides a nucleic acid encoding any of the above coronavirus S protein antibodies or the antigen-binding fragments thereof, or any one of the chains thereof. In one embodiment, provided is a vector comprising the nucleic acid. In one embodiment, the vector is an expression vector. In one embodiment, provided is a host cell comprising the nucleic acid or the vector. In one embodiment, the host cell is eukaryotic. In another embodiment, the host cell is selected from a yeast cell, a mammalian cell (e.g., a CHO cell or HEK293 cell), and other cells suitable for preparing an antibody or an antigen-binding fragment thereof. In another embodiment, the host cell is prokaryotic.

In one embodiment, the present invention provides one or more vectors comprising the nucleic acid. In one embodiment, the vector is an expression vector, such as a eukaryotic expression vector. The vector includes, but is not limited to, a virus, a plasmid, a cosmid, a λ phage or a yeast artificial chromosome (YAC).

Once the expression vector or DNA sequence has been prepared for expression, the expression vector can be transfected or introduced into suitable host cells. Various techniques can be used for this purpose, for example, calcium phosphate precipitation, protoplast fusion, retroviral transduction, viral transfection, electroporation, lipid-based transfection, biolistics, or other conventional techniques.

Methods and conditions for incubating the resulting transfected cells and for isolating the resulting antibody molecules are known to those skilled in the art and may be varied or optimized according to the particular expression vector and the particular mammalian host cell used based on the present description and methods known in the art.

Additionally, cells having stably incorporated DNA into chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be connected directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising the polynucleotide of the present invention. In some embodiments, provided is a host cell comprising the expression vector of the present invention. In some embodiments, the host cell is selected from a yeast cell, a mammalian cell, and other cells suitable for preparing an antibody. Suitable host cells comprise prokaryotic microorganisms, such as *E*. *coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK 293 or 293F cells), 293 cell, baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), Chinese hamster ovary cell (CHO cell), CHOS cell, NSO cell, and myeloma cell line such as Y0, NSO, P3X63 and Sp2/0. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO cell or an HEK 293 cell.

### V. Production and purification of coronavirus S protein antibody of the present invention

In one embodiment, the present invention provides a method for preparing a coronavirus S protein antibody, wherein the method comprises culturing a host cell comprising a nucleic acid encoding the coronavirus S protein antibody or an expression vector comprising the nucleic acid under a condition suitable for expressing the nucleic acid encoding the coronavirus S protein antibody, and optionally isolating the coronavirus S protein antibody. In a certain embodiment, the method further comprises isolating the coronavirus S protein antibody from the host cell (or host cell culture medium).

To recombinantly produce the coronavirus S protein antibody of the present invention, a nucleic acid encoding the coronavirus S protein antibody of the present invention is first isolated, and the nucleic acid is inserted into a vector for further cloning and/or expression in a host cell. Such nucleic acids can be easily isolated and sequenced by using conventional procedures, for example, by using an oligonucleotide probe that is capable of specifically binding to the nucleic acid encoding the coronavirus S protein antibody of the present invention. The coronavirus S protein antibody of the present invention prepared as described herein can be purified by known prior art, such as high performance liquid chromatography, ion exchange chromatography, gel electrophoresis, affinity chromatography, size exclusion chromatography, and the like. The actual conditions used to purify a particular protein also depend on factors such as net charge, hydrophobicity and hydrophilicity, and these will be apparent to those skilled in the art. The purity of the coronavirus S protein antibody of the present invention can be determined by any one of a variety of well-known analytical methods including size exclusion chromatography, gel electrophoresis, high performance liquid chromatography, and the like.

### VI. Activity assay of coronavirus S protein antibody of the present invention

The coronavirus S protein antibody provided herein can be identified, screened, or characterized for its physical/chemical properties and/or biological activity through a variety of assays known in the art.

The coronavirus S protein antibody of the present invention can be tested for its binding activity to the coronavirus S protein by known methods such as ELISA. Exemplary methods are disclosed herein.

The present invention further provides an assay for identifying coronavirus S protein antibodies having biological activities. The biological activities may include, for example, blocking the binding of the coronavirus S protein to ACE2 on the cell surface.

### VII. Pharmaceutical composition and pharmaceutical preparation

In some embodiments, the present invention provides a composition comprising at least one of any of the coronavirus S protein antibodies described herein, preferably the composition is a pharmaceutical composition. In some embodiments, the composition of the present invention comprises at least two antibodies or antigen-binding fragments of the present invention. For example, the composition of the present invention comprises two antibodies or antigen-binding fragments of the present invention and a pharmaceutically acceptable carrier.

In one embodiment, the composition of the present invention comprises two antibodies or antigen-binding fragments of the present invention in a molar ratio of 1:(0.5-1) and a pharmaceutically acceptable carrier. For example, the composition of the present invention comprises two antibodies or antigen-binding fragments of the present invention in a molar ratio of 1:0.5 and a pharmaceutically acceptable carrier; the composition of the present invention comprises two antibodies or antigen-binding fragments of the present invention in a molar ratio of 1:1 and a pharmaceutically acceptable carrier.

In one embodiment, the composition further comprises a pharmaceutical supplementary material.

In one embodiment, the composition (e.g., the pharmaceutical composition) comprises the coronavirus S protein antibody of the present invention and a combination of one or more additional therapeutic agents (e.g., an anti-infective active agent or a small molecule drug). The anti-infective active agent or the small molecule drug is any anti-infective active agent or small molecule drug used to treat, prevent or ameliorate a coronavirus infection in a subject, including, but not limited to, remdesivir, ribavirin, oseltamivir, zanamivir, hydroxychloroquine, interferon-α2b, analgesics, azithromycin, and corticosteroids. In the context of the present invention, the coronavirus infection includes an infection caused by a coronavirus (including, but not limited to, 2019-nCoV and SARS-CoV).

In some embodiments, the pharmaceutical composition or the pharmaceutical preparation of the present invention comprises a suitable pharmaceutical supplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible. The pharmaceutical carrier suitable for use in the present invention can be sterile liquid, such as water and oil, including petroleum, or oil of an animal, vegetable or a synthetic source, e.g., peanut oil, soybean oil, mineral oil, and sesame oil. Water is a preferred carrier when the pharmaceutical composition is administered intravenously. Saline solutions, aqueous dextrose and glycerol solutions can also be used as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. For use and application of the excipients, see Handbook of Pharmaceutical Excipients, 5th Ed., R. C. Rowe, P. J. Seskey and S. C. Owen, Pharmaceutical Press, London, Chicago. The composition may further comprise a small quantity of wetting agent, emulsifier, or pH buffer, if desired. The compositions may be in the form of a solution, a suspension, an emulsion, a tablet, a pill, a capsule, a powder, a sustained release preparation, and the like. Oral preparations may comprise standard pharmaceutical carriers and/or excipients such as pharmaceutical-grade mannitol, lactose, starch, magnesium stearate and saccharin.

The pharmaceutical preparation, preferably in the form of a lyophilized preparation or an aqueous solution, comprising the coronavirus S protein antibody of the present invention can be prepared by mixing the coronavirus S protein antibody disclosed herein of desired purity with one or more optional pharmaceutical supplementary materials (*Remington's Pharmaceutical Sciences,* 16^{th} Ed., Osol, A. ed. (1980)).

The pharmaceutical composition or preparation of the present invention may further comprise more than one active ingredient required by a particular indication treated, preferably those having complementarity activities without adversely affecting one another. For example, it is desirable to further provide additional anti-infective active ingredients, such as other antibodies, anti-infective active agents, small molecule drugs, or immunomodulatory agents. The active ingredients are suitably combined in an amount effective for an intended purpose.

A sustained release preparation can be prepared. Suitable examples of the sustained release preparation include a semipermeable matrix of a solid hydrophobic polymer comprising the coronavirus S protein antibody of the present invention. The matrix is in the form of a shaped article, such as a film or a microcapsule.

### VIII. Combination product or kit

In some embodiments, the present invention further provides a combination product comprising the coronavirus S protein antibody or the antigen-binding fragment thereof of the present invention, or further comprising one or more additional therapeutic agents (e.g., an anti-infective active agent, a small molecule drug, or an immunomodulatory agent, etc.).

In some embodiments, two or more of the ingredients in the combination product may be administered to a subject in combination sequentially, separately or simultaneously.

In some embodiments, the present invention further provides a kit comprising the coronavirus S protein antibody, the pharmaceutical composition, or the combination product of the present invention, and optionally a package insert directing administration.

In some embodiments, the present invention further provides a pharmaceutical product comprising the coronavirus S protein antibody, the pharmaceutical composition, or the combination product of the present invention, optionally further comprising a package insert directing administration.

### IX. Use of coronavirus S protein antibody of the present invention in prevention and/or treatment

The present invention provides a method for preventing a coronavirus-related disease or disorder in a subject, comprising administering to the subject the antibodies of the present invention or a combination of the antibodies.

Subjects at risk of suffering from a coronavirus-related disease include those who are in contact with an infected individual or are otherwise exposed to the coronavirus. The prophylactic agent can be administered prior to the manifestation of symptomatic characteristics of the coronavirus-related disease, so as to arrest the disease, or optionally, delay the progression of the disease.

The present invention further provides a method for treating a coronavirus-related disease in a patient. In one embodiment, the method involves administering to the patient with the disease the antibodies of the present invention that neutralize a coronavirus or a combination of the antibodies.

In some embodiments, provided is a method for treating a coronavirus infection in a patient, comprising administering an antibody selected from the group consisting of antibody molecules P3-11, P5-22, P10-20, P14-37, P14-44, P15-16, and P23-29, or an antigen-binding fragment thereof.

In some embodiments, provided is a method for treating a coronavirus infection in a patient, comprising administering an antibody of the group consisting of antibody molecules P5-22 and P14-44, or an antigen-binding fragment thereof.

In some embodiments, provided is a method for treating a coronavirus infection in a patient, comprising administering an antibody of the group consisting of antibody molecules P5-22 and P14-44 in a molar ratio of 1:1, or an antigen-binding fragment.

In some embodiments, the antibody or the antigen-binding fragment thereof of the present invention can cross-neutralize both human and animal infectious coronavirus isolates.

In some embodiments, the antibody or the antigen-binding fragment thereof of the present invention is administered within the first 24 hours after a coronavirus infection.

### X. Methods and compositions for diagnosis and detection of coronavirus

In some embodiments, any of the coronavirus S protein antibodies provided herein can be used to detect the presence of a coronavirus in a biological sample.

The term "detection" or "detect" as used herein includes quantitative and qualitative detections, and exemplary detections may involve immunohistochemistry, immunocytochemistry, flow cytometry (e.g., FACS), magnetic beads complexed with antibody molecules, and ELISA assay.

In one embodiment, provided is a coronavirus S protein antibody for use in a diagnostic or detection method. In another aspect, provided is a method for detecting the presence of a coronavirus in a biological sample. In certain embodiments, the method comprises detecting the presence of a coronavirus S protein in a biological sample. In certain embodiments, the method comprises contacting the biological sample with the coronavirus S protein antibody as described herein under a condition that allows the coronavirus S protein antibody to bind to the coronavirus S protein, and detecting whether a complex is formed by the coronavirus S protein antibody and the coronavirus S protein. The formation of the complex indicates the presence of a coronavirus. The method may be an *in vitro* or *in vivo* method.

Exemplary diagnostic assays for coronavirus include, for example, contacting a sample obtained from a patient with the anti-coronavirus S protein of the present invention, wherein a detectable label or reporter molecule is used to label the anti-coronavirus S protein or as a capture ligand to selectively isolate the coronavirus from the patient sample. Alternatively, an unlabeled anti-coronavirus S protein may be used in diagnostic applications in combination with a secondary antibody that is itself detectably labeled. The detectable label or reporter molecule may be a radioisotope such as ³H, ¹⁴C, ³²P, ³⁵S or ¹²⁵I, a fluorescent or chemiluminescent moiety such as fluorescein isothiocyanate or rhodamine, or an enzyme such as alkaline phosphatase, β-galactosidase, horseradish peroxidase or luciferase. Specific exemplary assays that can be used to detect or measure a coronavirus in a sample include enzyme-linked immunosorbent assay (ELISA), radioimmunoassay (RIA), and fluorescence-activated cell sorting (FACS).

The sample that can be used in the coronavirus diagnostic assay according to the present invention includes any biological sample available from a patient that comprises a coronavirus spike protein or a fragment thereof in an amount detectable under normal or physiological conditions. In some embodiments, the biological sample is blood, serum, a pharyngeal swab, a lower respiratory tract sample (e.g., tracheal secretion, tracheal aspirate, alveolar lavage fluid), or other samples of biological origin. Generally, the level of coronavirus spike protein in a specific sample obtained from a healthy patient (e.g., a patient not afflicted with a coronavirus-related disease) will be measured to initially establish a baseline or standard coronavirus level. The baseline level of coronavirus may then be compared with a level of coronavirus measured in a sample obtained from an individual suspected of having a coronavirus-related condition or a symptom associated with the condition.

The antibody specific for the coronavirus spike protein may not comprise other markers, or it may comprise an N-terminal or C-terminal marker. In one embodiment, the marker is biotin. In a binding assay, the position of the marker (if present) can determine the orientation of the peptide relative to the surface to which it is bound. For example, if the surface is coated with avidin, a peptide comprising N-terminal biotin will be oriented such that the C-terminal moiety of the peptide is directed away from the surface.

### XI. Sequences of exemplary anti-coronavirus S protein antibodies of the present invention

**Table 1. VH and VL sequences of antibodies (underlined sequences are CDR sequences)**

| Antibody Clone No. | VH/VL | Sequences |
|---|---|---|
| P5-22 | VH (DNA) | GAGGTGCAGCTGGTGGAGTCTGGGGGAGGCTTGGTCAAGCCTGGAG GGTCCCTGAGACTCTCCTGTGCAGCCTCTGGATTCACCTTCAGGGAC TACGACATCATCTGGATCCGCCAGGCTCCAGGGAAGGGGCTGGAGT |
| | | GGGTTTCATACATTAGTCGTAGTGGTAGTACCATATACTACTCAGAC TCTGTGAGGGGCCGATTCACCATCTCCAGGGACAACGCCAAGAATT CAGTGTATCTGCAAATGAACAGCCTGAGAGCCGAGGACACGGCCGT GTATTACTGTGCGAGAGATTTCGGGTTTGAGGGACCCCGTATGGAC GTCTGGGGCCAAGGGACCACGGTCACCGTCTCCTCA (SEQ ID NO:1) |
| | VH (amino acid) | EVQLVESGGGLVKPGGSLRLSCAASGFTFRDYDIIWIRQAPGKGLEWVS YISRSGSTIYYSDSVRGRFTISRDNAKNSVYLQMNSLRAEDTAVYYCAR DFGFEGPRMDVWGQGTTVTVSS (SEQ ID NO:2) |
| | VL (DNA) | GACATCCAGATGACCCAGTCTCCATCCTCCCTGTCTGCATCTGTAGG AGACAGAGTCACCATCACTTGCCAGGCGAGTCAGGACATTAAGAAC TATTTAAATTGGTATCAGCAGAAACCAGGGAAAGCCCCTAAGCTCC TGATCTACGATGCATCCAATTTGGAAACAGGGGTCCCATCAAGGTTC AGTGGAAGTGGATCTGGGACAGATTTTACTTTCACCATCAGCAGCCT GCAGCCTGAAGATATTGCAACATATTACTGTCAACAGTTTGATAATC TCCCGATCACCTTCGGCCAAGGGACACGACTGGAGATTAAA (SEQ ID NO:3) |
| | VL (amino acid) | DIQMTQSPSSLSASVGDRVTITCQASQDIKNYLNWYQQKPGKAPKLLIY DASNLETGVPSRFSGSGSGTDFTFTISSLQPEDIATYYCQQFDNLPITFGQ GTRLEIK (SEQ ID NO:4) |
| P14-44 | VH (DNA) | CAGGTGCAGCTGGTGCAGTCTGGGGCTGAGGTGAAGAAGCCTGGGG CCTCAGTGAAGGTTTCCTGCAAGGCATCTGGATACATTTTCACCAGC TATTCTATGCACTGGGTGCGACAGGCCCCTGGACAAGGGCTTGAGT GGATGGGAACAATCAAGCCTAGTGATGATAGCACAAACTACGCACA GAAGTTCCAGGGCAGAGTCTCCATGACCAGGGACACGTCCACGAGC ACAGTCTACATGGAGCTGAGCAGCCTGAGATATGAGGACACGGCCG TGTATTACTGTGCGAGAGAGGCCCGGGGATATTATGATAGAAGTGG TTATTACCACCCGGGTTACTTTGACTACTGGGGCCAGGGAACCCTGG TCACCGTCTCCTCA (SEQ ID NO:5) |
| | VH (amino acid) | QVQLVQSGAEVKKPGASVKVSCKASGYIFTSYSMHWVRQAPGQGLEW MGTIKPSDDSTNYAQKFQGRVSMTRDTSTSTVYMELSSLRYEDTAVYY CAREARGYYDRSGYYHPGYFDYWGQGTLVTVSS (SEQ ID NO:6) |
| | VL (DNA) | CAGTCTGTGCTGACTCAGCCTGCCTCCGTGTCTGGGTCTCCTGGACA GTCGATCACCATCTCCTGCACTGGAACCAGCAGTGACGTTGGTGGTT ATAACTTTGTCTCCTGGTACCAACAGCACCCAGGCAAAGCCCCCAA |
| | | ACTCATGATTTATGAGGTCAGTGATCGGCCCTCAGGGGTTTCTAGTC GCTTCTCTGGCTCCAAGTCTGGCAACACGGCCTCCCTGACCATCTCT GGGCTCCAGGCTGAGGACGAGGCTGATTATTACTGCTTCTCATATAC AACCAGCACCACTTGGGTGTTCGGCGGAGGGACCAAGCTGACCGTC CTA (SEQ ID NO:7) |
| | VL (amino acid) | QSVLTQPASVSGSPGQSITISCTGTSSDVGGYNFVSWYQQHPGKAPKLM IYEVSDRPSGVSSRFSGSKSGNTASLTISGLQAEDEADYYCFSYTTSTTW VFGGGTKLTVL (SEQ ID NO:8) |
| P15-16 | VH (DNA) | CAGGTGCAGCTGGTGGAGTCTGGGGGAGGCTTGGTACAGCCTGGCA GGTCCCTGACACTCTCCTGTGCAGCCTCTGGATTCACCTTTGATGATT ATGCCATGCACTGGGTCCGGCAAGCTCCAGGGAAGGGCCTGGAGTG GGTCTCAGGTATTAGTTGGAATAGTGGTACCATAGACTATGCGGACT CTGTGAAGGGCCGATTCACCATCTCCAGAGACAACGCCAAGAACTCC CTGTATCTGCAAATGAACAGTCTGAGAGCTGAGGACACGGCCTTGTA TTACTGTGCAACCCTCCCACGTGAACGTGCATATAGTGGCTACGATC GAATCTTCTACACTATAGACGTCTGGGGCCAAGGGACCACGGTCACC GTCTCCTCA (SEQ ID NO: 9) |
| | VH (amino acid) | QVQLVESGGGLVQPGRSLTLSCAASGFTFDDYAMHWVRQAPGKGLEW VSGISWNSGTIDYADSVKGRFTISRDNAKNSLYLQMNSLRAEDTALYYC ATLPRERAYSGYDRIFYTIDVWGQGTTVTVSS (SEQ ID NO: 10) |
| | VL (DNA) | GACATCCAGATGACCCAGTCTCCATCCTCCCTGTCTGCATCTGTAGG AGACAGAGTCACCATCACTTGCCGGGCAAGTCAGAGCATTAGCACCT ATTTAAATTGGTATCAGCAGAAACCAGGGAAAGCCCCTAAGCTCCTG ATCTATGCTGCATCCAGTTTGCAAAGTGGGGTCCCATCAAGGTTCAG TGGCAGTGGATCTGGGACAGATTTCACTCTCACCATCAGCAGT CTGCAACCTGAAGATTTTGCAACCTACTACTGTCAACAGAGTTACAG TACCCCTCTCACTTTCGGCGGAGGGACCAA GGTGGAAATCAAA (SEQ ID NO: 11) |
| | VL (amino acid) | DIQMTQSPSSLSASVGDRVTITCRASQSISTYLNWYQQKPGKAPKLLIYA ASSLQSGVPSRFSGSGSGTDFTLTISS LQPEDFATYYCQQSYSTPLTFGGGTKVEIK (SEQ ID NO: 12) |
| P10-20 | VH (DNA) | CAGGTGCAGCTGGTGGAGTCTGGGGGAGGCTTGGTCAAGCCTGGAG GGTCCCTGAGACTCTCCTGTGCAGCCTCTGGATTCACCTTCAGTGACT ACTACATGAGCTGGATCCGCCAGGCTCCAGGGAAGGGGCTGGAGTG |
| | | GGTTTCATACATTAGTATTAGTAGTACTTACACAAACTACGCAGACT CTGTGAAGGGCCGATTCACCATCTCCAGAGACAACGCCAAGAACTC ACTGTATCTGCAAATGAACAGCCTGAGAGCCGAGGACACGGCCGTG TATTACTGTGTGATAGCAGCAGCTGGTAAGGGTCACTACTACTACGG TATGGACGTCTGGGGCCAAGGGACCACGGTCACCGTCTCCTCA (SEQ ID NO: 13) |
| | VH (amino acid) | QVQLVESGGGLVKPGGSLRLSCAASGFTFSDYYMSWIRQAPGKGLEWV SYISISSTYTNYADSVKGRFTISRDNAKNSLYLQMNSLRAEDTAVYYCVI AAAGKGHYYYGMDVWGQGTTVTVSS (SEQ ID NO: 14) |
| | VL (DNA) | GACATCCAGATGACCCAGTCTCCATCCTCCCTGTCTGCATCTGTAGG AGACAGAGTCACCATCACTTGCCAGGCGAGTCAGGACATTAGCAAC TATTTAAATTGGTATCAGCAGAAACCAGGGAAAGCCCCTAAGCTCCT GATCTACGATGCATCCAGTTTGGAAACAGGGGTCCCATCAAGGTTCA GTGGAAGTGGATCTGGGACAGATTTTACTTTCACCATCAGCAGC CTGCAGCCTGAAGATATTGCAACATATTACTGTCAACAGTATGATAA TCTCCCGCTCACTTTCGGCGGAGGGACCAA GGTGGAGATCAAA (SEQ ID NO: 15) |
| | VL (amino acid) | DIQMTQSPSSLSASVGDRVTITCQASQDISNYLNWYQQKPGKAPKLLIY DASSLETGVPSRFSGSGSGTDFTFTISSLQPEDIATYYCQQYDNLPLTFGG GTKVEIK (SEQ ID NO: 16) |
| P14-37 | VH (DNA) | GAGGTGCAGCTGGTGGAGTCTGGGGGAGGCTTGGTAAAGCCTGGGG GGTCCCTTAGACTCTCCTGTGCCGCCTCTGGATTCACTTTCAGTAACG CCTGGATGAGCTGGGTCCGCCAGGCTCCAGGGAAGGGCCTGGAGTG GGTTGGCCGTATTAAAAGGAATAGCGATGGTGGGACAACAGACCAC GCTGCACCCGTGACAGGCAGATTCATCATCTCAAGAGATGATTCAAA AAACACGCTGTATCTGCAAATGAACAGCCTGAGAACCGAGGACACA GCCGTCTATTACTGTACCACAGATCTGGATACTGTAGTTCGGCGAGT TGTTATAACCGATCATGATGCTTTTGATATCTGGGGCCAAGGGACAA TGGTCACCGTCT CTTCA (SEQ ID NO: 17) |
| | VH (amino acid) | EVQLVESGGGLVKPGGSLRLSCAASGFTFSNAWMSWVRQAPGKGLEW VGRIKRNSDGGTTDHAAPVTGRFIISRDDSKNTLYLQMNSLRTEDTAVY YCTTDLDTVVRRVVITDHDAFDIWGQGTMVTVSS (SEQ ID NO: 18) |
| | VL (DNA) | GACATCCAGATGACCCAGTCTCCATCCTCCCTATCTGCATCTGTAGG AGACAGAGTCACCATCACTTGCCGGGCAAGTCAGAGCATTAGCAGC |
| | | TTTTTAAATTGGTATCAGCAGAAAGCAGGGAAAGCCCCTAAGCTCCT GATCTATGCTGCATCCAGTTTGCAAAGTGGGGTCCCATCAAGGTTCA GTGGCAGTGGATCTGGGACAGATTTCAGTCTCACCATCAGCAATCTG CAACCTGAAGATTTTGCAACTTACTACTGTCAACAGAGTTACAGTAC CCCTTCGATCACCTTCGGCCAAGGGAC ACGACTGGAGATTAAA (SEQ ID NO: 19) |
| | VL (amino acid) | DIQMTQSPSSLSASVGDRVTITCRASQSISSFLNWYQQKAGKAPKLLIYA ASSLQSGVPSRFSGSGSGTDFSLTISNLQPEDFATYYCQQSYSTPSITFGQ GTRLEIK (SEQ ID NO: 20) |
| P23-29 | VH (DNA) | CAGGTGCAGCTGCAGGAGTCGGGCCCAGGACTGGTGAAGCCTTCGG AGACCCTGTCCCTCACCTGCACTGTCTCTGGTGACTCCATCAGCAGT AGTACTTACTACTGGGGCTGGATCCGCCAGCCCCCAGGGAAGGGGCT GGAGTGGATTGGGAGTATGTATTATGGTCGGAGTACCTACTACAACC CGTCCCTCAAGAGTCGAGTCACCGTATCCGTAGACACGTCCAAGAAC CAGTTGTCCCTGAAGGTGAGCTCTGTGACCGCCGCAGACACGGCTGT CTATTACTGTGCGAGACATCTGGGTGG CGTTGACTACTGGGGCCAGGGAACCCTGGTCACCGTCTCTTCA (SEQ ID NO: 21) |
| | VH (amino acid) | QVQLQESGPGLVKPSETLSLTCTVSGDSISSSTYYWGWIRQPPGKGLEWI GSMYYGRSTYYNPSLKSRVTVSVDTSKNQLSLKVSSVTAADTAVYYCA RHLGGVDYWGQGTLVTVSS (SEQ ID NO: 22) |
| | VL (DNA) | GACATCCAGATGACCCAGTCTCCATCCTCCCTGTCTGCATCTGTAGG AGACAGAGTCACCATCACTTGCCGGGCGAGTCAGGGCATTAGCAATT CTTTAGCCTGGTATCAGCAGGAACCAGGGAAAGCCCCTAAGCTCCTG CTCTATGCTGCATCCACATTGGAAAGTGGGGTCCCATCCAGGTTCAG TGGCAGTGGGTCTGGGGCGGATTCCACTCTCACCATCAGCAGCCTGC AGCCTGAAGATTTTGCAACTTATTACTGTCAACAGTATTATAGTGCCT CCGCGCTCACTTTCGGCGGAGGGAC CAAGGTGGAGATTAAA (SEQ ID NO: 23) |
| | VL (amino acid) | DIQMTQSPSSLSASVGDRVTITCRASQGISNSLAWYQQEPGKAPKLLLY AASTLESGVPSRFSGSGSGADSTLTISSLQPEDFATYYCQQYYSASALTF GGGTKVEIK (SEQ ID NO: 24) |
| P3-11 | VH (DNA) | CAGGTGCAGCTGGTGGAGTCTGGGGGAGGCTTGGTCCAGCCTGGGG GGTCCCTGAGACTCTCCTGTGCAGCCTCTGGAATCACCGTCAGTAGC |
| | | AACTATATGAGCTGGGTCCGCCAGGCTCCAGGGAAGGGGCTGGAAT GGGTCTCAGTTATTTATAGCGGTGGTAGCACATTCTACGCAGACCCC GTGAAGGGCAGACTCACCATCTCCAGAGACAATTCCAAGAACACGC TGTATCTTCAAATGAACAGTGTGAGAGTCGAGGACACGGCTGTGTAT TACTGTGCGAGAGGAGAAGGATGGGAGCTGCCATTTGACTACTGGG GCCAGGGAGCCCTGGTCACCGTCTCCTCA (SEQ ID NO: 25) |
| | VH (amino acid) | QVQLVESGGGLVQPGGSLRLSCAASGITVSSNYMSWVRQAPGKGLEW VSVIYSGGSTFYADPVKGRLTISRDNSKNTLYLQMNSVRVEDTAVYYC ARGEGWELPFDYWGQGALVTVSS (SEQ ID NO: 26) |
| | VL (DNA) | CAGTCTGAGCTGACTCAGCCTCCCTCCGCGTCCGGGTCTCCTGGACA GTCAGTCACCATCTCCTGCACTGGAACCAGCAGTGACGTTGGTGGTA ATAAATATGTCTCCTGGTACCAACAGCACCCAGGCAAAGCCCCCAAA CTCATAATTTATGAGGTCAGTAAGCGGCCCTCAGGGGTCCCTGATCG CTTCTCTGGCTCCAAGTCTGGCAACACGGCCTCCCTGACCGTCTCTGG GCTCCAGGCTGAGGATGAGGCTGATTATCACTGCAGCTCATATGCAG GCAGCAACAATTTGGTGTTCGGCGG AGGGACCAAGCTGACCGTCCTA (SEQ ID NO: 27) |
| | VL (amino acid) | QSALTQPPSASGSPGQSVTISCTGTSSDVGGNKYVSWYQQHPGKAPKLII YEVSKRPSGVPDRFSGSKSGNTASLTVSGLQAEDEADYHCSSYAGSNNL VFGGGTKLTVL (SEQ ID NO: 28) |

**Table 2. CDR sequences of heavy chain variable regions of antibodies**

| Antibody | SEQ ID NO | HCDR1 (AbM numbering) | SEQ ID NO | HCDR2 (Kabat numbering) | SEQ ID NO | HCDR3 (Kabat numbering) |
|---|---|---|---|---|---|---|
| P5-22 | 29 | GFTFRDYDII | 30 | YISRSGSTIYYSDSVRG | 31 | DFGFEGPRMDV |
| P14-44 | 35 | GYIFTSYSM H | 36 | TIKPSDDSTNYAQKFQG | 37 | EARGYYDRSGYYHPGYF DY |
| P15-16 | 41 | GFTFDDYAM H | 42 | GISWNSGTIDYADSVKG | 43 | LPRERAYSGYDRIFYTID V |
| P10-20 | 47 | GFTFSDYYM S | 48 | YISISSTYTNYADSVKG | 49 | AAAGKGHYYYGMDV |
| P14-37 | 53 | GFTFSNAWM S | 54 | RIKRNSDGGTTDHAAPVT G | 55 | DLDTVVRRVVITDHDAF DI |
| P23-29 | 59 | GDSISSSTYY WG | 60 | SMYYGRSTYYNPSLKS | 61 | HLGGVDY |
| P3-11 | 65 | GITVSSNYM S | 66 | VIYSGGSTFYADPVKG | 67 | GEGWELPFDY |

**Table 3. CDR sequences of light chain variable regions of antibodies**

| Antibody | SEQ ID NO | LCDR1 (Kabat numbering) | SEQ ID NO | LCDR2 (Kabat numbering) | SEQ ID NO | LCDR3 (Kabat numbering) |
|---|---|---|---|---|---|---|
| P5-22 | 32 | QASQDIKNYLN | 33 | DASNLET | 34 | QQFDNLPIT |
| P14-44 | 38 | TGTSSDVGGYNFVS | 39 | EVSDRPS | 40 | FSYTTSTTWV |
| P15-16 | 44 | RASQSISTYLN | 45 | AASSLQS | 46 | QQSYSTPLT |
| P10-20 | 50 | QASQDISNYLN | 51 | DASSLET | 52 | QQYDNLPLT |
| P14-37 | 56 | RASQSISSFLN | 57 | AASSLQS | 58 | QQSYSTPSIT |
| P23-29 | 62 | RASQGISNSLA | 63 | AASTLES | 64 | QQYYSASALT |
| P3-11 | 68 | TGTSSDVGGNKYVS | 69 | EVSKRPS | 70 | SSYAGSNNLV |

The following examples are described to assist in understanding the present invention. The examples are not intended to be and should not be interpreted in any way as limiting the protection scope of the present invention.

### Examples

The present invention as generally described herein will be more easily understood by reference to the following examples, which are provided by way of illustration and are not intended to limit the scope of the present invention. These examples are not intended to indicate that the following experiments are all or only experiments performed.

### Example 1. Sorting of Single B Cells

10 mL of whole blood was obtained from a patient recovered from a novel coronavirus infection and placed in a heparin collection tube. Isolation was performed by density gradient centrifugation using a lymphocyte isolation solution, followed by B cell isolation using a B cell isolation kit (EasySep^{™} Human B Cell Enrichment Kit, STEMCELL, Cat. No. 19054). Sorting of single B cells was performed on BD FACSAria II by binding fluorescein-labeled S proteins to the isolated B cells using Alexa Fluor^{™} 488 kit (Invitrogen, Cat. No. A20181), Alexa Fluor^{™} 647 kit (Invitrogen, Cat. No. A20186), SARS-CoV-2 S1 protein (Aero, Cat. No. S1N-C52H3), and SARS-CoV-2 S protein trimer (Aero, Cat. No. SPN-C52H8) according to the manufacturer's instructions. The sorted single B cells were placed in a 96-well plate containing an HEPES lysis buffer (10% NP-40, no RNase) at one cell/well.

### Example 2. Cloning of Single Human B cells

The 96-well cell culture plate obtained in Example 1 was transferred from -80 °C to room temperature and subjected to RT-PCR. The heavy and light chain cDNAs of the antibodies were synthesized using the reagents and reaction volumes as follows.

| Reagent | Volume per well (about 6 µL) | Volume per 96-well plate (+10% additional) |
|---|---|---|
| Random hexamer (150 ng/µL) | 3 µL | 330 µL |
| dNTPs (100 mM) | 0.2 µL | 22 µL |
| Superscript IV | 0.25 µL | 27.5 µL |
| Nuclease-free water | 2.55 µL | 280.5 µL |

The genes of the heavy chain and light chain variable regions of the antibodies were amplified by nested PCR using the above heavy and light chain cDNAs of the antibodies as templates, primers positioned in the 5' end Leader and FR1 regions as upstream primers, and primers positioned in the constant and FR4 regions of the antibodies as downstream primers.

After the PCR was completed, agarose gel electrophoresis was performed, and PCR products of about 400 bp with light and heavy chains which can be paired were sequenced directly. The sequencing results were analyzed and aligned using MEGA7 software, and the appropriate antibody variable region sequences were used for the subsequent construction of antibody plasmids.

### Example 3. Construction and Expression of Antibody Expression Plasmids

The gene segments of the heavy chain and light chain variable regions of the antibodies were amplified by PCR. The gene segments of the heavy chain and light chain variable regions were separately ligated to a pcDNA3.1 vector by homologous recombinase from Nanjing Vazyme Biotech Co., Ltd. (Exnase^{®}II, Cat. No. C112-01), where an IgG1 subclass was selected as the constant region so as to obtain a light chain plasmid and a heavy chain plasmid.

The light chain plasmid and the heavy chain plasmid of the same antibody were then mixed in a molar ratio of 1:1, and transfected into HEK293 cells by polyethyleneimine (PEI) (Polysciences, Cat. No. 23966). After 5-7 days of culture, when the cell viability was below 60%, the cell culture supernatant was collected and purified by a Protein A affinity column to obtain a monoclonal antibody.

### Example 4. Binding Experiment of Antibodies to Novel Coronavirus (nCov) S Proteins

nCoV S (ACRO, SPN-C52H4) at a concentration of 1 µg/mL was applied onto one 96-well microplate at 100 µL per well, and the plate was left to stand overnight at 4 °C.

After the plate was washed in a plate washer (300 µL × 3), 300 µL of PBST + 5% BSA was added for blocking, and the resulting mixture was left to stand at room temperature for 1 h.

After the plate was washed in the plate washer (300 µL × 3), the antibody prepared in Example 3 was added (i.e. 100 µL of the antibody sample was added to each well). The antibody was diluted in a 3-fold gradient starting at 10 nM (12 concentrations, lateral dilution) and placed in a horizontal shaker at 300 rpm for reaction at room temperature for 1.5 h.

After the plate was washed in the plate washer (300 µL × 3), 100 µL of an HRP-conjugated goat anti-human IgG Fc secondary antibody diluted at a ratio of 1:5000 was added, and the resulting mixture was placed in a horizontal shaker at 300 rpm for reaction at room temperature for 35 min.

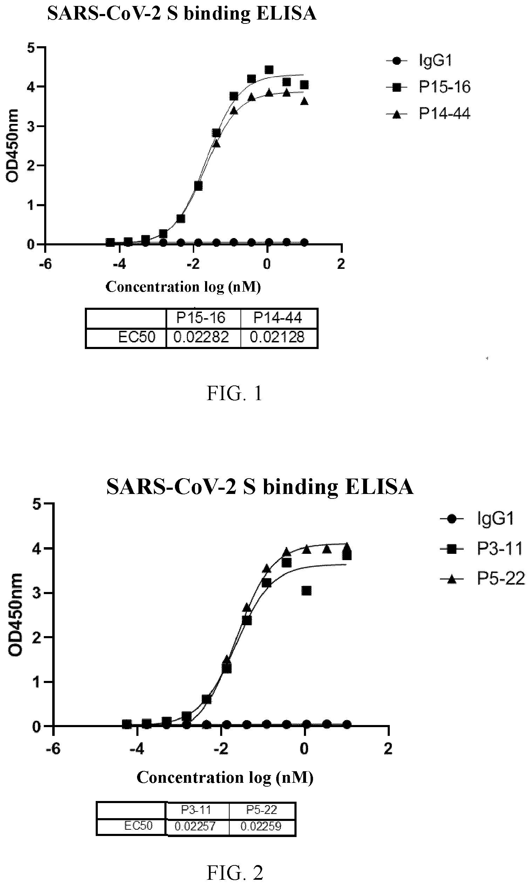

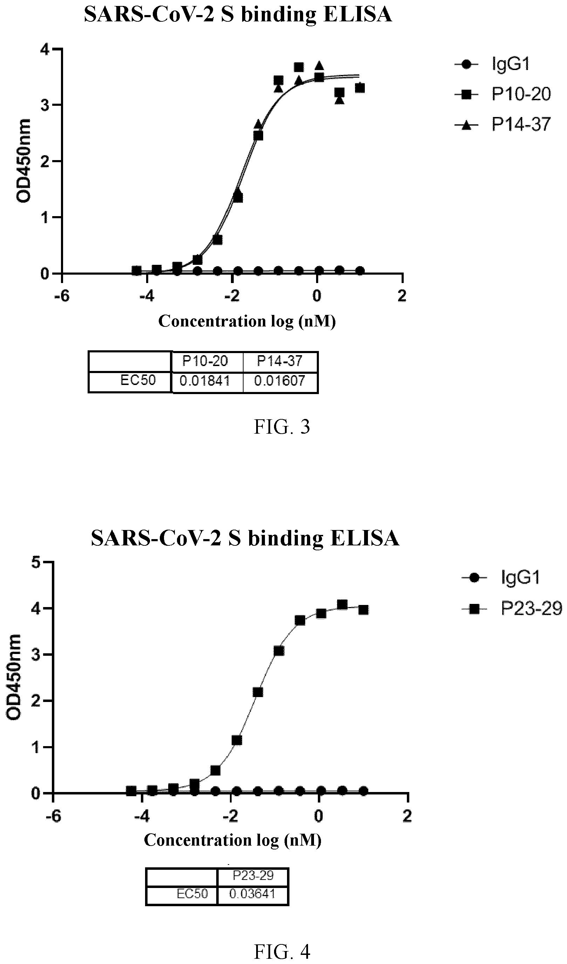

After the plate was washed twice in the plate washer, 100 µL of TMB was added for color development (the TMB liquid was placed in the dark at room temperature for 30 min in advance for later use), 100 µL of a terminating solution was added to terminate the reaction after 2 min of color development in the dark at room temperature, and the OD450 values were read by a microplate reader. The results are shown in FIGs. 1-4.

As can be seen from FIGs. 1-4, the candidate antibody molecules P3-11, P5-22, P10-20, P14-37, P14-44, P15-16, and P23-29 specifically bound to SARS-CoV-2 S protein with EC₅₀ values of 0.02257 nM, 0.02259 nM, 0.01841 nM, 0.01607 nM, 0.02128 nM, 0.02282 nM, and 0.03641 nM, respectively, and all of the antibody molecules had a strong specific binding ability to the SARS-CoV-2 S protein.

Example 5. Experiment of Blocking Binding of Novel Coronavirus (nCov) S Proteins to ACE2 by Antibodies ACE2-Fc (ACRO, AC2-H5257) at a concentration of 1 µg/mL was applied onto one 96-well microplate, and the plate was left to stand overnight at 4 °C.

After the plate was washed in a plate washer (300 µL × 3), 300 µL of PBST + 5% BSA was added to block the plate, and the resulting mixture was left to stand at room temperature for 1 h.

The plate was washed in the plate washer (300 µL × 3) for later use;
In addition, 2200 ng/mL Biotin-ncov RBD (KACTUS, COV-VM4BDB) was added to one new 96-well plate at 10 µL per well, and then an antibody sample was added at 100 µL per well. The antibody was diluted in a 3-fold gradient starting at 900 nM (12 concentrations, lateral dilution), with the final concentration of the Biotin-antigen being 200 ng/mL. The resulting mixture was placed in a horizontal shaker at 300 rpm for reaction at room temperature for 15 min. The reaction solution in this plate was transferred to a BSA-blocked, washed plate, and placed in a horizontal shaker at 300 rpm for reaction at room temperature for 1.5 h.

After the plate was washed in the plate washer (300 µL × 3), 100 µL of an eBioscience Avidin HRP secondary antibody (Invitrogen, Cat. No. 18-4100-51) diluted at a ratio of 1:2000 was added, and the resulting mixture was placed in a horizontal shaker at 300 rpm for reaction at room temperature for 35 min.

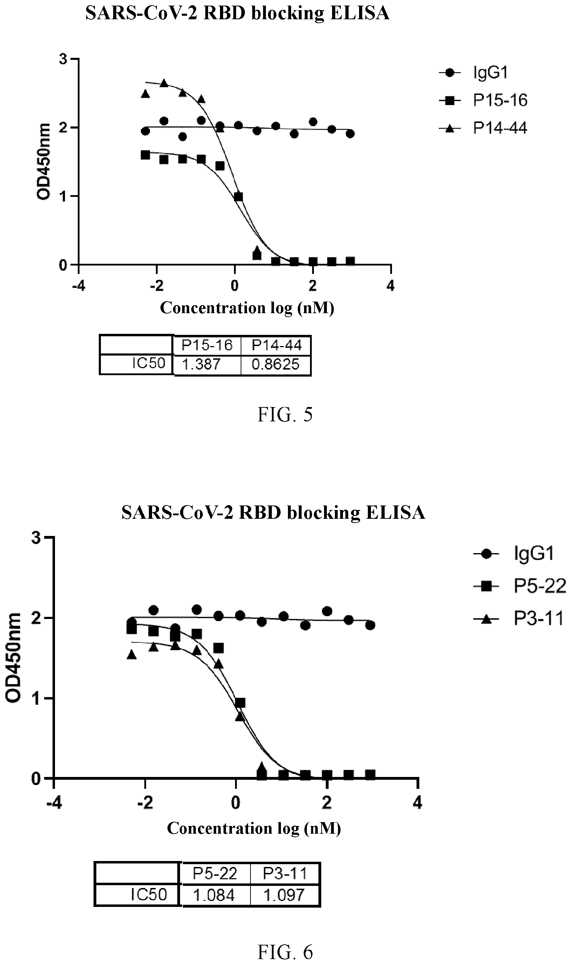

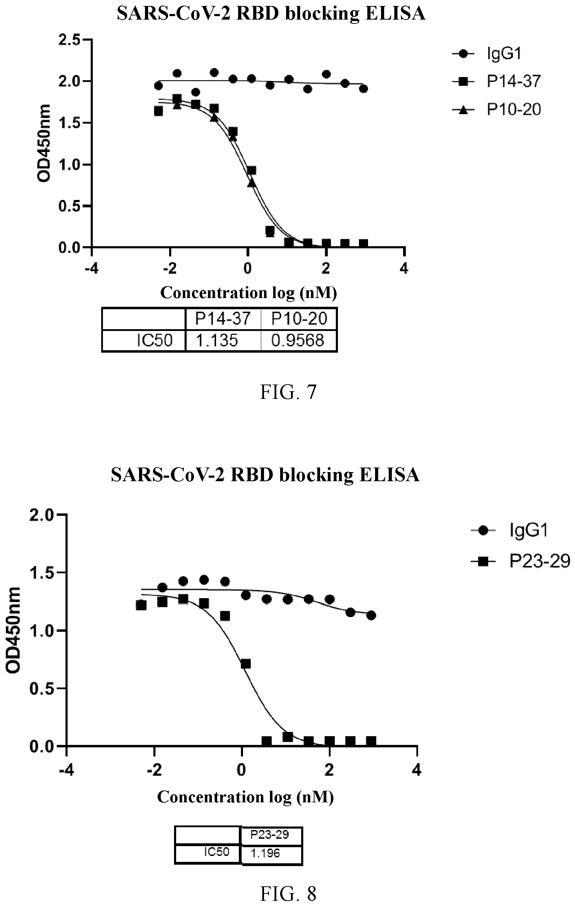

After the plate was washed twice in the plate washer, 100 µL of TMB was added for color development (the TMB liquid was placed in the dark at room temperature for 30 min in advance for later use), 100 µL of a terminating solution was added to terminate the reaction after 5min of color development in the dark at room temperature, and the OD450 values were read by a microplate reader. The results are shown in FIGs. 5-8.

As can be seen from FIGs. 5-8, the candidate antibody molecules P3-11, P5-22, P10-20, P14-37, P14-44, P15-16, and P23-29 blocked the binding of the SARS-CoV-2 S protein to its receptor ACE2 with IC₅₀ values of 1.097nM, 1.084 nM, 0.9568 nM, 1.135 nM, 0.8625 nM, 1.387 nM, and 1.196 nM, respectively, and all of the antibody molecules had a strong blocking effect on the binding of the SARS-CoV-2 S protein to its receptor ACE2.

### Example 6. Neutralization Experiment of Pseudotyped SARS-CoV-2 Virus

The candidate antibody molecules in Example 4 and Example 5, as well as an isotype human IgG1 antibody as a control, were prepared. A pseudovirus expressing SARS-CoV-2 S protein was purchased (Genscript, Cat. No. C628AFE090, 1.5 × 10⁸ IFU/mL). A DMEM cell culture medium was prepared, containing 89% DMEM high glucose medium, 10% FBS, and 1% GLUTAMAX.

Information on the reagents used in the experiment is shown in the table below.

| Name | Manufacturer & brand | Cat. No. | Batch No. |
|---|---|---|---|
| Lenti-X^{™} Concentrator | Takara | 631232 | 1905232A |
| pLV-luci | Inovogen Tech. Co. | No. VL3612 | N/A |
| Opti-MEM | Gibco | 31985-070 | 2003628 |
| PEIpro | Polyplus | 115-100 | 29011C1A |
| DMEM high glucose medium | GIBCO, USA | 11965-092 | 2120395 |
| FBS, New Zealand | HYCLONE, USA | SH30406.05 | DB0543 |
| DPBS | Thermo Fisher, USA | 14190136 | 2152835 |
| Pancreatin 0.25% Trypsin-EDTA (1X) | GIBCO, USA | 25200072 | 1930154 |
| Bio-Glo Luciferase Assay System | PROMEGA, USA | G7940 | 0000391249 |

Information on the materials used in the experiment is shown in the table below.

| Name | Manufacturer & brand | Cat. No. | Batch No. |
|---|---|---|---|
| T75 simple culture bottle | NUNC, Denmark | 156499 | 8440321 |
| 50 mL centrifuge tube | NUNC, Denmark | 339652 | 14AF348118 |

| Name | Manufacturer & brand | Cat. No. | Batch No. |
|---|---|---|---|
| 15 mL centrifuge tube | NUNC, Denmark | 339650 | I4AF347116 |
| 96-well round-bottom culture plate | CORNING, USA | CLS3799-50EA | 35517005 |
| 96-well white cell culture plate | NUNC, Denmark | 136101 | 157137 |
| 50 mL loading tank (sterile) | JET, USA | LTT001050 | 180825-139 |
| 25 mL pipette | NUNC, Denmark | 170357N | HH05008 |
| C-Chip disposable hemocytometer | INCYTO, Korea | DHC-N01 | NK06A2641H |
| 10 mL pipette | NUNC, Denmark | 170356N | FK04028 |

The neutralization experiment of SARS-CoV-2 pseudovirus was conducted as follows.

Solution preparation: A cryopreserved Bio-Glo Luciferase Assay System was thawed at 4 °C in the dark, and the solution in the Bio-Glo Luciferase Assay System was mixed with a powder in a biosafety cabinet in the dark. The resulting mixture was subpackaged into 11 mL centrifuge tubes, with 10 mL in each tube, and the tubes were stored in a freezer at -40 °C in the dark.

Preparation of cells: The cultured HEK293/ACE2 cells (Genescript R10232004) were digested and resuspended in a DMEM cell culture medium at a density of 6.67 × 10⁴ cells/mL. The cell suspension was added to a white-bottom 96-well plate at 150 µL per well, with 1 × 10⁴ cells in each well, and the plate was covered with a lid and incubated in a CO₂ incubator for 8-10 h.

Antibody dilution: The antibody was diluted in a 3-fold gradient starting at 120 nM (alternatively, diluted in a 3-fold gradient starting at 40 nM) with a DMEM cell culture medium to give a final volume of 50 µL.

Incubation with pseudovirus: Pseudovirus (S envelop) was melted in a water bath at 37 °C, added to the diluted antibody at 10 µL per well, and incubated on ice for 1 h. The incubated mixed system of the antibody and the pseudovirus was sequentially added to the cells cultured in the white-bottom 96-well plate at 50 µL per well such that the antibody had a final concentration of 30 nM, and the antibody was diluted in a 3-fold gradient (alternatively, diluted in a 3-fold gradient starting at 10 nM). The white-bottom 96-well plate was placed back into the CO₂ incubator and incubated for another 48 h.

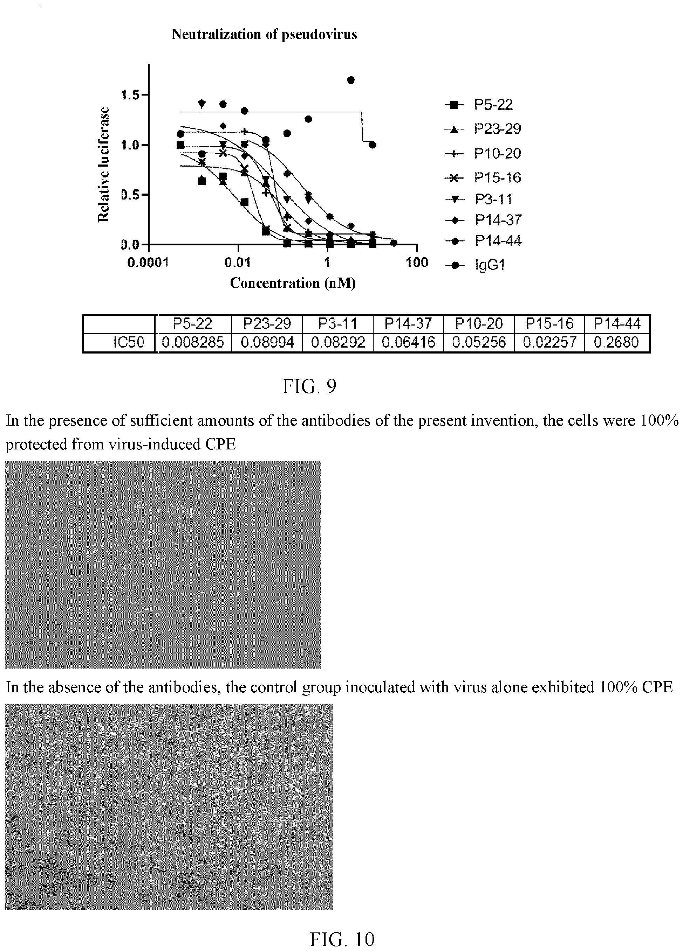

Detection: After 48 h, one aliquot of the cryopreserved Bio-Glo Luciferase Assay System was taken out and thawed at 4 °C in the dark. The white-bottom 96-well plate incubated for 48 h was taken out from the incubator, 100 µL of the medium was carefully aspirated, and a Bio-Glo Luciferase Reagent solution was added at 100 µL per well. The detection was rapidly performed using a microplate reader. The results are shown in FIG. 9.

As can be seen from FIG. 9, the candidate antibody molecules P3-11, P5-22, P10-20, P14-37, P14-44, P15-16, and P23-29 blocked the binding of the pseudovirus to the HEK293/ACE2 cells with IC₅₀ values of 0.08292 nM, 0.008285 nM, 0.05256 nM, 0.06416 nM, 0.2680 nM, 0.02257 nM, and 0.08994 nM, respectively, preliminarily indicating that the antibody molecules have a strong blocking effect on the binding of the pseudovirus S protein to the receptor ACE2 on the cell surface at the cellular level.

### Example 7. Neutralization Experiment of SARS-CoV-2 Euvirus

Experimental virus strains: SARS-CoV-2 euvirus was a strain isolated from a clinical case of novel coronavirus-infected pneumonia in Jiangsu, China.

Cell line: VERO-E6 cells belong to the green monkey kidney cell line, naturally expressing ACE2. Green monkey ACE2 is highly conserved with human ACE2, with a sequence homology of 95%. In this example, the VERO-E6 cell line was selected to replace the cell line expressing human ACE2 for the experiment. Experimental method:
VERO-E6 cells were infected with SARS-CoV-2 euvirus. 5 days after infection, the 50% tissue culture (in this example, cells) infection dose (TCID₅₀) was calculated by the Karber method.

Verification of activity of the neutralizing antibodies was performed by using a trace virus inhibition experimental method. A fixed amount of virus (100 TCID₅₀) was mixed with equal volumes of antibodies at different dilutions to infect VERO-E6 cells that had grown into monolayers, and replicate wells were set for each antibody dilution for detection. A normal cell control was also set up in the experiment.

After inoculation, the cells were observed for CPE (cytopathic effect) daily for 3-5 consecutive days. The CPE results are shown in FIG. 10.

As can be seen from FIG. 10, in the presence of sufficient amounts of the antibodies of the present invention, the cells were 100% protected from virus-induced CPE. In the absence of the antibodies, the control group inoculated with virus alone exhibited 100% CPE.

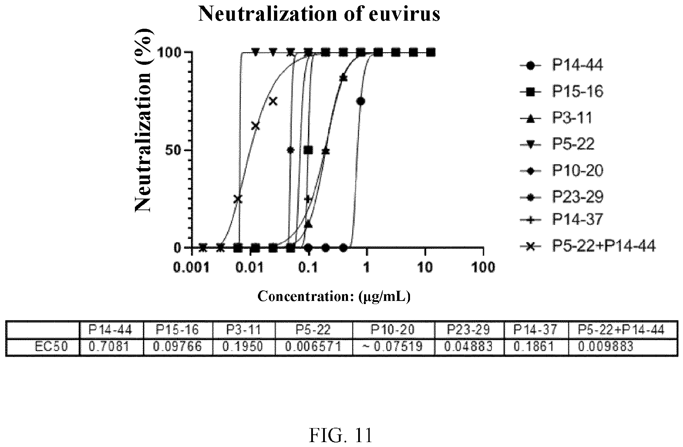

The results of the neutralization of SARS-CoV-2 euvirus on the fourth day by the candidate antibody molecules in Example 4 and Example 5 are shown in FIG. 11.

As can be seen from FIG. 11, the candidate antibody molecules P3-11, P5-22, P10-20, P14-37, P14-44, P15-16, and P23-29 neutralized the infection of the VERO-E6 cells by the SARS-CoV-2 euvirus with EC₅₀ values of 0.1950 µg/mL, 0.006571 µg/mL, about 0.07519 µg/mL, 0.1861 µg/mL, 0.7081 µg/mL, 0.09766µg/mL, and 0.04883 µg/mL, respectively, indicating that the antibody molecules have a strong blocking effect on the binding of the SARS-CoV-2 euvirus S protein to the receptor ACE2 on the cell surface at the cellular level.

Similarly, the candidate antibody molecules P5-22 and P14-44 were combined (in a molar ratio of 1:1) and then subjected to a neutralization experiment with the euvirus. The results show that the combination neutralized the infection of VERO-E6 cells by SARS-CoV-2 euvirus with an EC₅₀ value of 0.009883 µg/mL (FIG. 11), indicating that the combination of the antibodies of the present invention can also strongly block the binding of the SARS-CoV-2 euvirus S protein to the receptor ACE2 on the cell surface at the cellular level. The combination of the antibodies prevents a viral mutation from potentially escaping from the blocking by an antibody.

The exemplary embodiments of the present invention have been described above. It should be understood by those skilled in the art that these contents are merely exemplary, and various other replacements, adaptations and modifications can be made within the scope of the present invention. Therefore, the present invention is not limited to the specific embodiments listed herein.

## Claims

1. An isolated anti-coronavirus S protein antibody or an antigen-binding fragment, comprising:
(a) 3 CDRs in an amino acid sequence of a heavy chain variable region set forth in SEQ ID NO: 2 and 3 CDRs in an amino acid sequence of a light chain variable region set forth in SEQ ID NO: 4; or a CDR variant having no more than 3 amino acid residue substitutions relative to any one of the 6 CDRs;
(b) 3 CDRs in an amino acid sequence of a heavy chain variable region set forth in SEQ ID NO: 6 and 3 CDRs in an amino acid sequence of a light chain variable region set forth in SEQ ID NO: 8; or a CDR variant having no more than 3 amino acid residue substitutions relative to any one of the 6 CDRs;
(c) 3 CDRs in an amino acid sequence of a heavy chain variable region set forth in SEQ ID NO: 10 and 3 CDRs in an amino acid sequence of a light chain variable region set forth in SEQ ID NO: 12; or a CDR variant having no more than 3 amino acid residue substitutions relative to any one of the 6 CDRs;
(d) 3 CDRs in an amino acid sequence of a heavy chain variable region set forth in SEQ ID NO: 14 and 3 CDRs in an amino acid sequence of a light chain variable region set forth in SEQ ID NO: 16; or a CDR variant having no more than 3 amino acid residue substitutions relative to any one of the 6 CDRs;
(e) 3 CDRs in an amino acid sequence of a heavy chain variable region set forth in SEQ ID NO: 18 and 3 CDRs in an amino acid sequence of a light chain variable region set forth in SEQ ID NO: 20; or a CDR variant having no more than 3 amino acid residue substitutions relative to any one of the 6 CDRs;
(f) 3 CDRs in an amino acid sequence of a heavy chain variable region set forth in SEQ ID NO: 22 and 3 CDRs in an amino acid sequence of a light chain variable region set forth in SEQ ID NO: 24; or a CDR variant having no more than 3 amino acid residue substitutions relative to any one of the 6 CDRs; or
(g) 3 CDRs in an amino acid sequence of a heavy chain variable region set forth in SEQ ID NO: 26 and 3 CDRs in an amino acid sequence of a light chain variable region set forth in SEQ ID NO: 28; or a CDR variant having no more than 3 amino acid residue substitutions relative to any one of the 6 CDRs.

2. The isolated antibody or the antigen-binding fragment according to claim 1, comprising:
(a) an HCDR1 set forth in SEQ ID NO: 29 or a variant of the HCDR1 set forth in SEQ ID NO: 29 having no more than 3 amino acid residue substitutions, an HCDR2 set forth in SEQ ID NO: 30 or a variant of the HCDR2 set forth in SEQ ID NO: 30 having no more than 3 amino acid residue substitutions, and an HCDR3 set forth in SEQ ID NO: 31 or a variant of the HCDR3 set forth in SEQ ID NO: 31 having no more than 3 amino acid residue substitutions; and an LCDR1 set forth in SEQ ID NO: 32 or a variant of the LCDR1 set forth in SEQ ID NO: 32 having no more than 3 amino acid residue substitutions, an LCDR2 set forth in SEQ ID NO: 33 or a variant of the LCDR2 set forth in SEQ ID NO: 33 having no more than 3 amino acid residue substitutions, and an LCDR3 set forth in SEQ ID NO: 34 or a variant of the LCDR3 set forth in SEQ ID NO: 34 having no more than 3 amino acid residue substitutions;
(b) an HCDR1 set forth in SEQ ID NO: 35 or a variant of the HCDR1 set forth in SEQ ID NO: 35 having no more than 3 amino acid residue substitutions, an HCDR2 set forth in SEQ ID NO: 36 or a variant of the HCDR2 set forth in SEQ ID NO: 36 having no more than 3 amino acid residue substitutions, and an HCDR3 set forth in SEQ ID NO: 37 or a variant of the HCDR3 set forth in SEQ ID NO: 37 having no more than 3 amino acid residue substitutions; and an LCDR1 set forth in SEQ ID NO: 38 or a variant of the LCDR1 set forth in SEQ ID NO: 38 having no more than 3 amino acid residue substitutions, an LCDR2 set forth in SEQ ID NO: 39 or a variant of the LCDR2 set forth in SEQ ID NO: 39 having no more than 3 amino acid residue substitutions, and an LCDR3 set forth in SEQ ID NO: 40 or a variant of the LCDR3 set forth in SEQ ID NO: 40 having no more than 3 amino acid residue substitutions;
(c) an HCDR1 set forth in SEQ ID NO: 41 or a variant of the HCDR1 set forth in SEQ ID NO: 41 having no more than 3 amino acid residue substitutions, an HCDR2 set forth in SEQ ID NO: 42 or a variant of the HCDR2 set forth in SEQ ID NO: 42 having no more than 3 amino acid residue substitutions, and an HCDR3 set forth in SEQ ID NO: 43 or a variant of the HCDR3 set forth in SEQ ID NO: 43 having no more than 3 amino acid residue substitutions; and an LCDR1 set forth in SEQ ID NO: 44 or a variant of the LCDR1 set forth in SEQ ID NO: 44 having no more than 3 amino acid residue substitutions, an LCDR2 set forth in SEQ ID NO: 45 or a variant of the LCDR2 set forth in SEQ ID NO: 45 having no more than 3 amino acid residue substitutions, and an LCDR3 set forth in SEQ ID NO: 46 or a variant of the LCDR3 set forth in SEQ ID NO: 46 having no more than 3 amino acid residue substitutions;
(d) an HCDR1 set forth in SEQ ID NO: 47 or a variant of the HCDR1 set forth in SEQ ID NO: 47 having no more than 3 amino acid residue substitutions, an HCDR2 set forth in SEQ ID NO: 48 or a variant of the HCDR2 set forth in SEQ ID NO: 48 having no more than 3 amino acid residue substitutions, and an HCDR3 set forth in SEQ ID NO: 49 or a variant of the HCDR3 set forth in SEQ ID NO: 49 having no more than 3 amino acid residue substitutions; and an LCDR1 set forth in SEQ ID NO: 50 or a variant of the LCDR1 set forth in SEQ ID NO: 50 having no more than 3 amino acid residue substitutions, an LCDR2 set forth in SEQ ID NO: 51 or a variant of the LCDR2 set forth in SEQ ID NO: 51 having no more than 3 amino acid residue substitutions, and an LCDR3 set forth in SEQ ID NO: 52 or a variant of the LCDR3 set forth in SEQ ID NO: 52 having no more than 3 amino acid residue substitutions;
(e) an HCDR1 set forth in SEQ ID NO: 53 or a variant of the HCDR1 set forth in SEQ ID NO: 53 having no more than 3 amino acid residue substitutions, an HCDR2 set forth in SEQ ID NO: 54 or a variant of the HCDR2 set forth in SEQ ID NO: 54 having no more than 3 amino acid residue substitutions, and an HCDR3 set forth in SEQ ID NO: 55 or a variant of the HCDR3 set forth in SEQ ID NO: 55 having no more than 3 amino acid residue substitutions; and an LCDR1 set forth in SEQ ID NO: 56 or a variant of the LCDR1 set forth in SEQ ID NO: 56 having no more than 3 amino acid residue substitutions, an LCDR2 set forth in SEQ ID NO: 57 or a variant of the LCDR2 set forth in SEQ ID NO: 57 having no more than 3 amino acid residue substitutions, and an LCDR3 set forth in SEQ ID NO: 58 or a variant of the LCDR3 set forth in SEQ ID NO: 58 having no more than 3 amino acid residue substitutions;
(f) an HCDR1 set forth in SEQ ID NO: 59 or a variant of the HCDR1 set forth in SEQ ID NO: 59 having no more than 3 amino acid residue substitutions, an HCDR2 set forth in SEQ ID NO: 60 or a variant of the HCDR2 set forth in SEQ ID NO: 60 having no more than 3 amino acid residue substitutions, and an HCDR3 set forth in SEQ ID NO: 61 or a variant of the HCDR3 set forth in SEQ ID NO: 61 having no more than 3 amino acid residue substitutions; and an LCDR1 set forth in SEQ ID NO: 62 or a variant of the LCDR1 set forth in SEQ ID NO: 62 having no more than 3 amino acid residue substitutions, an LCDR2 set forth in SEQ ID NO: 63 or a variant of the LCDR2 set forth in SEQ ID NO: 63 having no more than 3 amino acid residue substitutions, and an LCDR3 set forth in SEQ ID NO: 64 or a variant of the LCDR3 set forth in SEQ ID NO: 64 having no more than 3 amino acid residue substitutions; or
(g) an HCDR1 set forth in SEQ ID NO: 65 or a variant of the HCDR1 set forth in SEQ ID NO: 65 having no more than 3 amino acid residue substitutions, an HCDR2 set forth in SEQ ID NO: 66 or a variant of the HCDR2 set forth in SEQ ID NO: 66 having no more than 3 amino acid residue substitutions, and an HCDR3 set forth in SEQ ID NO: 67 or a variant of the HCDR3 set forth in SEQ ID NO: 67 having no more than 3 amino acid residue substitutions; and an LCDR1 set forth in SEQ ID NO: 68 or a variant of the LCDR1 set forth in SEQ ID NO: 68 having no more than 3 amino acid residue substitutions, an LCDR2 set forth in SEQ ID NO: 69 or a variant of the LCDR2 set forth in SEQ ID NO: 69 having no more than 3 amino acid residue substitutions, and an LCDR3 set forth in SEQ ID NO: 70 or a variant of the LCDR3 set forth in SEQ ID NO: 70 having no more than 3 amino acid residue substitutions,
wherein, preferably, the isolated antibody or the antigen-binding fragment comprises:
(a) an HCDR1 set forth in SEQ ID NO: 29, an HCDR2 set forth in SEQ ID NO: 30, and an HCDR3 set forth in SEQ ID NO: 31; and an LCDR1 set forth in SEQ ID NO: 32, an LCDR2 set forth in SEQ ID NO: 33, and an LCDR3 set forth in SEQ ID NO: 34;
(b) an HCDR1 set forth in SEQ ID NO: 35, an HCDR2 set forth in SEQ ID NO: 36, and an HCDR3 set forth in SEQ ID NO: 37; and an LCDR1 set forth in SEQ ID NO: 38, an LCDR2 set forth in SEQ ID NO: 39, and an LCDR3 set forth in SEQ ID NO: 40;
(c) an HCDR1 set forth in SEQ ID NO: 41, an HCDR2 set forth in SEQ ID NO: 42, and an HCDR3 set forth in SEQ ID NO: 43; and an LCDR1 set forth in SEQ ID NO: 44, an LCDR2 set forth in SEQ ID NO: 45, and an LCDR3 set forth in SEQ ID NO: 46;
(d) an HCDR1 set forth in SEQ ID NO: 47, an HCDR2 set forth in SEQ ID NO: 48, and an HCDR3 set forth in SEQ ID NO: 49; and an LCDR1 set forth in SEQ ID NO: 50, an LCDR2 set forth in SEQ ID NO: 51, and an LCDR3 set forth in SEQ ID NO: 52;
(e) an HCDR1 set forth in SEQ ID NO: 53, an HCDR2 set forth in SEQ ID NO: 54, and an HCDR3 set forth in SEQ ID NO: 55; and an LCDR1 set forth in SEQ ID NO: 56, an LCDR2 set forth in SEQ ID NO: 57, and an LCDR3 set forth in SEQ ID NO: 58;
(f) an HCDR1 set forth in SEQ ID NO: 59, an HCDR2 set forth in SEQ ID NO: 60, and an HCDR3 set forth in SEQ ID NO: 61; and an LCDR1 set forth in SEQ ID NO: 62, an LCDR2 set forth in SEQ ID NO: 63, and an LCDR3 set forth in SEQ ID NO: 64; or
(g) an HCDR1 set forth in SEQ ID NO: 65, an HCDR2 set forth in SEQ ID NO: 66, and an HCDR3 set forth in SEQ ID NO: 67; and an LCDR1 set forth in SEQ ID NO: 68, an LCDR2 set forth in SEQ ID NO: 69, and an LCDR3 set forth in SEQ ID NO: 70.

3. The isolated antibody or the antigen-binding fragment according to claim 1 or 2, comprising:
(a) a heavy chain variable region and a light chain variable region, wherein the heavy chain variable region comprises an amino acid sequence of SEQ ID NO: 2 or a sequence having at least 90%, 91%, 92%, 93%, 94%, 95%, 96%, 97%, 98%, or 99% identity thereto, and the light chain variable region comprises an amino acid sequence of SEQ ID NO: 4 or a sequence having at least 90%, 91%, 92%, 93%, 94%, 95%, 96%, 97%, 98%, or 99% identity thereto;
(b) a heavy chain variable region and a light chain variable region, wherein the heavy chain variable region comprises an amino acid sequence of SEQ ID NO: 6 or a sequence having at least 90%, 91%, 92%, 93%, 94%, 95%, 96%, 97%, 98%, or 99% identity thereto, and the light chain variable region comprises an amino acid sequence of SEQ ID NO: 8 or a sequence having at least 90%, 91%, 92%, 93%, 94%, 95%, 96%, 97%, 98%, or 99% identity thereto;
(c) a heavy chain variable region and a light chain variable region, wherein the heavy chain variable region comprises an amino acid sequence of SEQ ID NO: 10 or a sequence having at least 90%, 91%, 92%, 93%, 94%, 95%, 96%, 97%, 98%, or 99% identity thereto, and the light chain variable region comprises an amino acid sequence of SEQ ID NO: 12 or a sequence having at least 90%, 91%, 92%, 93%, 94%, 95%, 96%, 97%, 98%, or 99% identity thereto;
(d) a heavy chain variable region and a light chain variable region, wherein the heavy chain variable region comprises an amino acid sequence of SEQ ID NO: 14 or a sequence having at least 90%, 91%, 92%, 93%, 94%, 95%, 96%, 97%, 98%, or 99% identity thereto, and the light chain variable region comprises an amino acid sequence of SEQ ID NO: 16 or a sequence having at least 90%, 91%, 92%, 93%, 94%, 95%, 96%, 97%, 98%, or 99% identity thereto;
(e) a heavy chain variable region and a light chain variable region, wherein the heavy chain variable region comprises an amino acid sequence of SEQ ID NO: 18 or a sequence having at least 90%, 91%, 92%, 93%, 94%, 95%, 96%, 97%, 98%, or 99% identity thereto, and the light chain variable region comprises an amino acid sequence of SEQ ID NO: 20 or a sequence having at least 90%, 91%, 92%, 93%, 94%, 95%, 96%, 97%, 98%, or 99% identity thereto;
(f) a heavy chain variable region and a light chain variable region, wherein the heavy chain variable region comprises an amino acid sequence of SEQ ID NO: 22 or a sequence having at least 90%, 91%, 92%, 93%, 94%, 95%, 96%, 97%, 98%, or 99% identity thereto, and the light chain variable region comprises an amino acid sequence of SEQ ID NO: 24 or a sequence having at least 90%, 91%, 92%, 93%, 94%, 95%, 96%, 97%, 98%, or 99% identity thereto; or
(g) a heavy chain variable region and a light chain variable region, wherein the heavy chain variable region comprises an amino acid sequence of SEQ ID NO: 26 or a sequence having at least 90%, 91%, 92%, 93%, 94%, 95%, 96%, 97%, 98%, or 99% identity thereto, and the light chain variable region comprises an amino acid sequence of SEQ ID NO: 28 or a sequence having at least 90%, 91%, 92%, 93%, 94%, 95%, 96%, 97%, 98%, or 99% identity thereto,
wherein, preferably, the isolated antibody or the antigen-binding fragment is a fully human antibody or antigen-binding fragment.

4. The isolated antibody or the antigen-binding fragment according to any one of claims 1 to 3, being an IgG1, IgG2, IgG3, or IgG4 antibody, preferably, an IgG1 or IgG4 antibody, and more preferably, a human IgG1 or human IgG4 antibody.

5. The isolated antibody or the antigen-binding fragment according to any one of claims 1 to 4, wherein the antigen-binding fragment is an Fab, an Fab', an F(ab')₂, an Fv, a single-chain Fv, a single-chain Fab, or a diabody.

6. The isolated antibody or the antigen-binding fragment according to any one of claims 1 to 5, wherein the coronavirus is SARS-CoV-2 virus.

7. An isolated nucleic acid, encoding the antibody or the antigen-binding fragment according to any one of claims 1 to 6.

8. A vector, comprising the nucleic acid according to claim 7, wherein preferably, the vector is an expression vector.

9. A host cell, comprising the nucleic acid according to claim 7 or the vector according to claim 8, wherein, preferably, the host cell is prokaryotic or eukaryotic, more preferably, the host cell is selected from an E. *coli* cell, a yeast cell, a mammalian cell, and other cells suitable for preparing the antibody or the antigen-binding fragment, and most preferably, the host cell is an HEK293 cell.

10. A method for preparing the antibody or the antigen-binding fragment according to any one of claims 1 to 6, comprising culturing the host cell according to claim 9 under a condition suitable for expressing a nucleic acid encoding the antibody or the antigen-binding fragment according to any one of claims 1 to 6, and optionally isolating the antibody or the antigen-binding fragment according to any one of claims 1 to 6 from the host cell or a culture medium.

11. A pharmaceutical composition, comprising the antibody or the antigen-binding fragment according to any one of claims 1 to 6 and a pharmaceutically acceptable carrier; preferably, comprising at least two antibodies or antigen-binding fragments according to any one of claims 1 to 6 and a pharmaceutically acceptable carrier; more preferably, comprising two antibodies or antigen-binding fragments according to any one of claims 1 to 6 in a molar ratio of 1:(0.5-1) and a pharmaceutically acceptable carrier; and most preferably, comprising the antibody or the antigen-binding fragment (a) and the antibody or the antigen-binding fragment (b) according to any one of claims 1 to 6 in a molar ratio of 1:1, and a pharmaceutically acceptable carrier.

12. Use of the antibody or the antigen-binding fragment according to any one of claims 1 to 6, or the pharmaceutical composition according to claim 11 in the preparation of a medicament for preventing and/or treating a coronavirus infection, wherein, for example, the coronavirus is SARS-CoV-2 virus.

13. A method for preventing and/or treating a coronavirus infection in a subject, comprising administering to the subject an effective amount of the antibody or the antigen-binding fragment according to any one of claims 1 to 6, or the pharmaceutical composition according to claim 11, wherein, for example, the coronavirus is SARS-CoV-2 virus.

14. A kit for detecting a coronavirus S protein in a sample, comprising the antibody or the antigen-binding fragment according to any one of claims 1 to 6 for conducting the following steps:
(a) contacting the sample with the antibody or the antigen-binding fragment according to any one of claims 1 to 6; and
(b) detecting the formation of a complex by the antibody or the antigen-binding fragment according to any one of claims 1 to 6 and the coronavirus S protein,
wherein, for example, the coronavirus is SARS-CoV-2 virus.
